# EUROPÄISCHE PATENTSCHRIFT

(11) **EP 3 747 345 B1**
(45) Veröffentlichungstag und Bekanntmachung des Hinweises auf die Patenterteilung: **28.06.2023**
(21) Anmeldenummer: 20177131.8
(22) Anmeldetag: 28.05.2020
(51) Int. Cl.: A61B 1/00, G02B 23/24, H04N 13/239, H04N 13/286, H04N 13/296

(54) **BILDGEBUNGSSYSTEM UND VERFAHREN ZUR BEOBACHTUNG**
IMAGING SYSTEM AND OBSERVATION METHOD
SYSTÈME D'IMAGERIE ET PROCÉDÉ D'OBSERVATION

(30) Priorität: 03.06.2019 DE 102019114817
(43) Veröffentlichungstag der Anmeldung: 09.12.2020
(73) Patentinhaber: Karl Storz SE & Co. KG, 78532 Tuttlingen (DE)
(72) Erfinder: Schoch, Dieter, 78532 Tuttlingen (DE); Zepf, Marius, 78532 Tuttlingen (DE)
(74) Vertreter: Witte, Weller & Partner Patentanwälte mbB

(56) Entgegenhaltungen:
- DE-A1-102017 219 621
- US-A1- 2014 323 801
- US-A1- 2017 188 792
- US-A1- 2018 042 453

## Beschreibung

Die vorliegende Offenbarung betrifft ein Stereo-Bildgebungssystem mit einem Beobachtungsinstrument mit einer Bilderfassungseinheit zur Erfassung erster Bilddaten und zweiter Bilddaten, welche zur Stereo-Beobachtung kombinierbar sind.

insbesondere betrifft die vorliegende Offenbarung ein medizinisches Bildgebungssystem. Solche Bildgebungssysteme dienen regelmäßig zur Beobachtung des menschlichen und/oder tierischen Körpers.

Zur Stereo-Beobachtung geeignete Bildgebungssysteme umfassen üblicherweise Beobachtungsinstrumente mit stereoskopischer Beobachtungsoptik sowie Stereo-Bildsensoren, also beispielsweise zwei zueinander versetzte Bildsensoren. Es gibt also zwei Beobachtungskanäle. Bildsensoren umfassen üblicherweise CCD-Sensoren, CMOS-Sensoren und Ähnliches. Bei Verwendung zweier zueinander versetzter Bildsensoren ist einer der beiden Bildsensoren dem rechten Auge (erster Beobachtungskanal) und der andere der beiden Bildsensoren dem linken Auge des Beobachters (zweiter Beobachtungskanal) zugeordnet.

Zur Stereo-Beobachtung geeignete Bildgebungssysteme werden zuweilen auch als 3D-Bildgebungssysteme bezeichnet. Es versteht sich, dass damit nicht unbedingt 3D-Daten erfasst werden. Das auf diese Weise generierte 3D-Bild erlaubt jedoch dem Beobachter eine Tiefenwahrnehmung.

Medizinische Bildgebungssysteme umfassen beispielsweise Systeme zur endoskopischen Beobachtung. Im Sinne der vorliegenden Offenbarung umfassen Systeme zur endoskopischen Bildbearbeitung Gestaltungen, bei denen ein sondenähnliches Instrument in natürliche oder künstlich geschaffene Körperöffnungen einführbar ist, um Bilder des Körperinneren zu erfassen. Dies umfasst auch sogenannte Videoendoskopie-Systeme.

Aus der US 2014/0323801 A1 ist ein chirurgisches Bildgebungssystem bekannt, das eine Bildgebungseinheit aufweist, die zur Bereitstellung von Mono-Signalen und zur Bereitstellung von Stereo-Signalen ausgebildet ist. Das Bildungssystem dient zur Überwachung chirurgischer Eingriffe mit chirurgischen Instrumenten. Das Bildgebungssystem ist zur Stereo-Erfassung unter Nutzung einer stereoskopischen Parallaxe zwischen zwei Bildsensoren ausgebildet. Bei bestimmten Betriebszuständen wird nur ein Mono-Signal ohne stereoskopische Darstellung genutzt.

Aus der US 2018/042453 A1 ist eine stereoskopische Endoskopievorrichtung bekannt. Aus der US 2017/188792 A1 ist ein teleoperatives medizinisches System bekannt, mit einer Bildgebungseineit, die mit einer fernbetätigten Baugruppe zur motorischen Steuerung gekoppelt ist.

Aus der US 9,848,758 B2 ist ein Instrument in Form eines Stereo-Endoskops bekannt, bei dem Bilddaten für den rechten Kanal und den linken Kanal korrigiert werden, und bei dem auf Basis einer Prüfung während der Montage des Endoskops Identifikationsinformationen gewonnen werden, die sicherstellen sollen, dass die Korrekturdaten tatsächlich auch für den rechten bzw. linken Kanal bereitgestellt werden.

Aus der WO 2010/105946 A1 ist ein Endoskop bekannt, mit einem Bildsensor und einem Inertialsensor am distalen Ende, wobei der Inertialsensor dazu ausgebildet ist, eine Verkippung des Bildsensors relativ zum Gravitationsfeld zu erfassen, wobei das Endoskop ferner eine Steuereinrichtung aufweist, wobei der Bildsensor ein Array von Bildpixeln umfasst, um Bilddaten zu erzeugen, wobei der Inertialsensor dazu ausgebildet ist, ein die Verkippung repräsentierendes Verkippungssignal zu erzeugen, und wobei die Steuereinrichtung dazu ausgebildet ist, das Verkippungssignal mit den Bilddaten unter Ersetzung eines Teils der Bilddaten durch das Verkippungssignal zu einem Kombinationssignal zu kombinieren.

Aus der DE 10 2017 219 621 A1 ist ein medizinisches Visualisierungssystem bekannt, umfassend ein Endoskop und ein Mikroskop, die in verschiedenen Orientierungen ausgerichtet sind und deren Sichtachsen schräg zueinander stehen können. Das Endoskop ist mit einem Bewegungssensor versehen, wobei ein Winkel der Sichtachse des Endoskops relativ zur Sichtachse des Mikroskops ermittelt wird. Es ist eine Anzeigevorrichtung vorgesehen, die ein erstes Bild auf Basis der Mikroskop-Daten und ein zweites Bild auf Basis der Endoskop-Daten anzeigt, wobei die Orientierung des zweiten Bildes in Abhängigkeit vom Winkel der Sichtachse des Endoskops ausgerichtet werden kann.

Aus der WO 2016/012248 A1 ist ein Stereoendoskop mit distal angeordneten Bildaufnehmern bekannt. Es werden Ausführungsformen mit gerader Blickrichtung und Ausführungsformen mit schräger Blickrichtung beschrieben.

Aus der US 7,108,657 B2 ist eine endoskopische Visualisierungsvorrichtung mit zwei Bildaufnehmern bekannt, die zueinander versetzt sind, wobei vorgeschlagen wird, die beiden Bildaufnehmer jeweils um eine Achse senkrecht zu deren Sensorebene drehbar zu gestalten. Auf diese Weise kann das erfasste Bild gedreht werden. Damit ist eine Bildaufrichtungsfunktion gegeben.

Aus der US 7,037,258 B2 ist ein Video-Endoskop mit Mitteln zur Bildaufrichtung bekannt. Das Endoskop ist als monoskopisches Instrument gestaltet. Es handelt sich folglich um ein Instrument mit einem Beobachtungskanal. Das Bild wird elektronisch gedreht bzw. aufgerichtet.

Aus der US 7,134,992 B2 ist ein weiteres Video-Endoskop mit Mitteln zur Bildaufrichtung bekannt. Es wird vorgeschlagen, das erfasste Bild in Abhängigkeit von einer ermittelten Orientierung des Instruments aufzurichten. Das Endoskop weist einen am distalen Ende angeordneten Bildsensor auf. Es handelt sich um ein monoskopisches Beobachtungsinstrument mit einem Beobachtungskanal. Zur Erfassung der Orientierung dienen Beschleunigungssensoren.

Ferner bezieht sich die vorliegende Offenbarung auf medizinische Bildgebungssysteme, die zur Beobachtung des Körpers von außerhalb des Körpers ausgebildet sind. Mit anderen Worten ist es bei diesen Systemen nicht vorgesehen, das Instrument zumindest teilweise in eine Körperöffnung einzuführen. Derartige Systeme, insbesondere deren Beobachtungsinstrumente, werden beispielsweise als sogenannte Exoskope bezeichnet. Ein solches Exoskop mit einer stereoskopischen Optik ist aus der US 10,122,897 B2 bekannt. Es wird vorgeschlagen, die optische Einheit drehbar zu gestalten, um das Bild des Instruments bedarfsweise aufzurichten. Damit kann die Orientierung der Stereobasis der stereoskopischen Optik angepasst werden, so dass auch bei geänderter Orientierung bzw. Position des Instruments eine stereoskopische Beobachtung ermöglicht ist.

Die vorliegende Offenbarung bezieht sich zumindest in beispielhaften Ausgestaltungen auf Bildgebungssysteme und Instrumente zur Weißlicht-Beobachtung. Dies umfasst die Erfassung von sichtbarem Licht, insbesondere von Teilen des elektromagnetischen Spektrums, die für das menschliche Auge sichtbar sind. Dies betrifft beispielsweise einen Bereich von 380 nm bis 750 nm (Nanometer). Dies ist jedoch nicht einschränkend zu verstehen.

insbesondere schließt dies nicht aus, dass ein offenbarungsgemäßes Bildgebungssystem auch zur Beobachtung im Infrarot oder Nahinfrarot-Bereich bzw. im Ultraviolett-Bereich Bildinformationen erfassen kann. Dies ist beispielsweise bei Instrumenten für PDD (Photodynamische Diagnostik) und/oder PDT (Photodynamische Therapie) sowie für Instrumente zur Fluoreszenz-Beobachtung vorstellbar.

Zumindest in wesentlichen Ausführungsformen soll dies jedoch keine radiologischen Bildgebungsverfahren umfassen (beispielsweise Radiografie, Computertomografie, Magnetresonanztomografie, Ultraschall-Beobachtung). Demgemäß liegt der Fokus der vorliegenden Offenbarung nicht auf Bildgebungssystemen für die Schnittbild-Beobachtung. Hingegen beziehen sich wesentliche Aspekte der vorliegenden Offenbarung auf die Beobachtung von Oberflächen oder oberflächennahen Objekten. Es versteht sich, dass gleichwohl kombinierte Bildgebungssysteme vorstellbar sind.

Bei der Nutzung von Beobachtungsinstrumenten der vorstehend beschriebenen Art ist es häufig erforderlich, diese zu drehen oder in anderer Weise zu bewegen, etwa um ihre Längsachse (Achse des Instrumentenschaftes oder der Beobachtungsoptik), um den beobachteten Bildausschnitt zu verändern. Dies ist insbesondere bei handgehaltenen bzw. handgeführten Instrumenten der Fall. Jedoch auch bei Instrumenten, die starr aufgenommen sind, können Bewegungsfreiheitsgrade vorgesehen sein, um das Instrument und dessen Beobachtungsoptik in der gewünschten Weise zu positionieren.

Bei Beobachtungsinstrumenten mit nur einem Bildsensor kann die Ausrichtung des Bildsensors in Bezug auf einen Referenzhorizont (definiert etwa durch ein Wiedergabegerät, beispielsweise einen Monitor) angepasst werden, indem das Bild mechanisch oder elektronisch gedreht wird. Auf diese Weise kann eine Bildaufrichtung durchgeführt werden. Im Sinne der vorliegenden Offenbarung ist unter dem Merkmal Bildaufrichtung eine Funktion zu verstehen, bei der die Orientierung (etwa die Richtungen oben, unten, rechts, links) des angezeigten Bildes weitgehend oder vollständig beibehalten wird, obwohl der Bildsensor bzw. die Beobachtungsoptik gedreht wird (im Sinne einer Rollbewegung). Diese Definition ist sinngemäß auch auf Beobachtungsinstrumente mit geneigter Blickrichtung zu übertragen, also beispielsweise auf Instrumente mit Objektiven, deren "Normale" nicht parallel zur Drehachse ist. Bei einem Objektiv handelt es sich bei der "Normalen" etwa um die optische Achse. Beispielsweise handelt es sich bei einem Bildsensor, der konzentrisch zum Objektiv ausgerichtet ist, bei der "Normalen" etwa um eine Senkrechte zur Sensorebene. Wesentlich für die Definition der Blickrichtung ist im Normalfall die optische Achse des Objektivs.

Es ist jedoch auch vorstellbar, das Bild eben nicht aufzurichten, so dass das angezeigte Bild bei einer Drehung oder Rollbewegung des Instruments mit rotiert. Entsprechende Wiedergabemodi können wahlweise ausgewählt werden.

Bei einem zur Stereo-Beobachtung gestalteten Instrument bringt die Bildaufrichtung verschiedene Herausforderungen mit sich. Hauptursache hierfür ist die sogenannte Stereobasis, also ein Versatz (Abstand bzw. Winkel) zwischen den beiden Beobachtungskanälen bzw. Bildsensoren, die für die Stereo-Beobachtung und Stereo-Wiedergabe kombiniert werden. Die Stereobasis ist idealerweise mit dem Referenzhorizont des Wiedergabegerätes ausgerichtet, idealerweise parallel oder nahezu parallel dazu. Der Referenzhorizont des Wiedergabegerätes entspricht üblicherweise einer Horizontalen. Ein Beobachter mit gerade ausgerichtetem Kopf richtet seine Augen (gedankliche Linie durch die beiden Augen) parallel hierzu aus.

Ein generelles Auslegungsziel für Bildgebungssysteme im medizinischen oder industriellen Umfeld besteht darin, den jeweiligen Sondenteil klein zu gestalten. Dies kann auch eine Miniaturisierung der Bilderfassungseinheit umfassen. Dies gilt beispielsweise für einen Durchmesser der Sonde bzw. der Objektivbaugruppe. Dieses Auslegungsziel ist umso wichtiger bei endoskopischen Instrumenten, die in der Neurochirurgie (Hirnchirurgie, Wirbelsäulenchirurgie, etc.) verwendet werden. Dies erschwert jedoch mechanische Lösungen für die Bildaufrichtung.

Allgemein ist es wünschenswert, Schaftbaugruppen endoskopischer Instrumente mit möglichst geringem Schaftdurchmesser zu gestalten. Dies kann das Trauma für den Patienten, beim Schaffen der Zugangsöffnung, minimieren. Daher gilt dieses Auslegungsziel allgemein für Instrumente für die minimalinvasive Chirurgie.

Gleichwohl gibt es auch bei möglichst klein (betreffend etwa den Schaftdurchmesser) bauenden Instrumenten Bedarf für stereoskopische Bilderfassung bzw. Beobachtung. Ein Ansatz für ein stereoskopisches Instrument, beispielsweise ein Endoskop, kann darin bestehen, zwei Bildsensoren am oder nahe dem distalen Ende des Instruments anzuordnen. Die beiden Bildsensoren sind regelmäßig nebeneinander angeordnet und haben daher Auswirkung auf den Gesamtdurchmesser des Instruments in diesem Bereich. Es wäre sehr aufwendig, in diesem distalen Bereich zusätzlich noch Vorkehrungen für eine mechanische Bildaufrichtung vorzusehen.

Gleichwohl werden auch derartige Instrumente häufig während der Nutzung um die Längsachse des Schaftes gedreht. Dies gilt insbesondere für Instrumente mit schräger Blickrichtung. Wie vorstehend bereits angedeutet, bringt dies jedoch bei der stereoskopischen Wahrnehmung Herausforderungen mit sich, zumindest sofern das Bild auch bei einer Rollbewegung aufgerichtet bleiben soll. Wenn im Umkehrschluss auf die Aufrichtung verzichtet wird, wird aus Sicht des Beobachters die Orientierung "im Bild" erschwert.

Instrumente mit nur einem Bildsensor, also mit nur einem Beobachtungskanal (monoskopische Instrumente) sind in dieser Hinsicht weniger problematisch. Das erfasste Bild kann sowohl mechanisch (tatsächlich von Hand oder über einen Aktuator) als auch elektronisch aufgerichtet (digitale Aufrichtung) werden. Die mechanische Aufrichtung umfasst eine Rotation des Sensors in Bezug auf den Schaft bzw. ein Lagerstück des Instruments. Die elektronische Bildaufrichtung umfasst bildverarbeitende Maßnahmen, so dass bei Bedarf sichergestellt werden kann, dass das Bild aufrecht wiedergegeben wird.

Vor diesem Hintergrund liegt der vorliegenden Offenbarung die Aufgabe zugrunde, ein Stereo-Bildgebungssystem mit einem Beobachtungsinstrument und ein Verfahren zur Stereo-Beobachtung anzugeben, die auch bei einer Rotation des Instruments bzw. der Bilderfassungseinheit des Instruments eine praktikabel wahrnehmbare Wiedergabe erlauben, wobei das wiedergegebene Bild bedarfsweise aufgerichtet werden kann. Gleichwohl soll in einem möglichst großen Rotationsbereich eine stereoskopische Darstellung (oder 3D-Darstellung) ermöglicht sein. Vorzugsweise ist eine intuitive Bedienung möglich. Vorzugsweise erlaubt das Bildgebungssystem bzw. das Verfahren in einem Zustand, in dem keine stereoskopische Erfassung und Wiedergabe sinnvoll ist, noch eine Grundfunktionalität, so dass zumindest ein Beobachtungskanal nutzbar ist.

Ferner soll im Rahmen der vorliegenden Offenbarung ein korrespondierendes Computerprogramm zur Steuerung eines Stereo-Bildgebungssystems angegeben werden.

Diese Aufgabe wird gemäß einem ersten Aspekt durch ein Stereo-Bildgebungssystem, insbesondere medizinisches Bildgebungssystem, nach Anspruch 1 gelöst.

Die Aufgabe der Erfindung wird auf diese Weise vollkommen gelöst.

Erfindungsgemäß wird nämlich einerseits die Fähigkeit zur Stereo-Beobachtung genutzt. Dies gilt jedenfalls in solchen Positionen (v.a. Drehlagen) der Bilderfassungseinheit, in denen die Orientierung der Stereobasis nicht oder nur in vertretbaren Grenzen von der Lagereferenz (etwa vom Horizont bzw. künstlichen Horizont) abweicht.

Wenn jedoch die vom Lagesensor bereitgestellten Signale betreffend die Orientierung (Drehlage bzw. Roll-Lage) ergeben, dass die Stereo-Funktionalität aufgrund der Verdrehung der Bilderfassungseinheit nicht mehr nutzbar ist, dann kann die Steuereinrichtung ein Mono-Signal (Nutzung nur eines Beobachtungskanals) ausgeben. Das kann dann der Fall sein, wenn sich der Bediener nicht mehr gut im Bild orientieren kann, beispielsweise bei der Steuerung dort sichtbarer chirurgischer Instrumente.

Die vorstehende Maßnahme hat den Vorteil, dass eine Bildaufrichtung, insbesondere eine elektronische Bildaufrichtung, ermöglicht wird, wobei in Abhängigkeit vom aktuellen Drehwinkel eine stereoskopische Wiedergabe auf Basis zweier Beobachtungskanäle oder eine monoskopische Wiedergabe auf Basis eines Beobachtungskanals möglich ist.

Dies bedeutet zwar, dass in bestimmten Orientierungen des Beobachtungsinstruments bzw. von dessen Bilderfassungseinheit nur eine zweidimensionale Wiedergabe ohne Tiefeneindruck genutzt wird. Gleichwohl kann auch diese Art der Wiedergabe zur Beobachtung genutzt werden.

Wenn jedoch über den Lagesensor ermittelt wird, dass die aktuelle Drehorientierung (Roll-Lage) der Bilderfassungseinheit eine stereoskopische Wiedergabe, also eine Wiedergabe mit Tiefeneindruck (3D-Wiedergabe) ermöglicht, dann kann auf stereoskopische Wiedergabe umgeschaltet werden. Hinsichtlich der Bildaufrichtung ist es vorstellbar, bei einer Verdrehung der Bilderfassungseinheit um etwa 180°die beiden Beobachtungskanäle für die Wiedergabe zu vertauschen und deren Bildinformationen jeweils um 180°zu drehen. Auch auf diese Weise kann zumindest für einen kleinen Drehwinkelbereich (bei etwa 180°) eine Bildaufrichtung auch bei stereoskopischer Wiedergabe realisiert werden.

Gemäß diesem Ansatz kann, zumindest in beispielhaften Ausgestaltungen, ein Instrument realisiert werden, bei dem einerseits die Stereo-Beobachtung und andererseits eine elektronische Bildaufrichtung möglich ist. Dies bezieht sich auf beispielhafte Ausführungsformen und ist nicht einschränkend zu verstehen.

Gleichwohl eignet sich dieser Ansatz in besonderer Weise für Instrumente mit kleinem Schaftdurchmesser, also für Instrumente für die minimalinvasive Chirurgie, insbesondere für die Neurochirurgie (Hirnchirurgie, Wirbelsäulenchirurgie). In beispielhaften Ausführungsformen handelt es sich bei dem Beobachtungsinstrument um ein medizinisches Beobachtungsinstrument. Je kleiner der Schaftdurchmesser, desto schwieriger wird es, mechanische Lösungen für die Bildaufrichtung zu implementieren.

Das Beobachtungsinstrument ist regelmäßig als Video-Endoskop oder Video-Exoskop gestaltet. Ein Video-Endoskop ist zur Beobachtung des Körperinneren ausgebildet und kann über eine natürliche oder künstliche Körperöffnung in das Körperinnere eingeführt werden. Ein Video-Exoskop ist zur Beobachtung des Körpers von außerhalb des Körpers ausgebildet. Die Instrumente können für die Humanmedizin und gegebenenfalls für die Veterinärmedizin ausgebildet sein. Gleichwohl sind auch industrielle Einsätze für Video-Endoskope und Video-Exoskope vorstellbar.

Die Lagereferenz, auf die sich das durch den Lagesensor ermittelte Lagesignal bezieht, kann beispielsweise ein Horizont (gegebenenfalls künstlicher Horizont) sein. Die Lagereferenz ergibt sich beispielsweise durch den Augenabstand (bzw. die Orientierung des zugehörigen Vektors) des Beobachters bei der Betrachtung des wiedergegebenen Bildes.

Beispielhaft umfasst die Bilderfassungseinheit zwei voneinander beabstandete Bildsensoren. Diese können parallel zueinander aber auch leicht relativ zueinander geneigt sein. Jedem der beiden Bildsensoren (rechts und links) ist einer von zwei Beobachtungskanälen zugeordnet. Die beiden Bildsensoren sind zur Stereo-Beobachtung eines Objekts in einer Objektebene miteinander kombinierbar. Die beiden Bildsensoren sind voneinander beabstandet und demgemäß an die Disparität der Augen eines Beobachters angepasst.

Diese Ausgestaltung ist nicht einschränkend zu verstehen. Allgemein ergibt sich die Stereobasis durch distale Aperturen der Objektiveinheit bzw. deren Abstand voneinander. Dies ist auch für die Lage der Bildsensoren bzw. deren Abstand maßgebend.

Im Falle einer Mono-Wiedergabe bzw. 2D-Beobachtung wird nur einer der beiden Kanäle, also nur einer der beiden Bildsensoren benutzt. Dies heißt nicht unbedingt, dass nur einer der beiden Bildsensoren tatsächlich ein Signal liefert. Jedoch wird zumindest bei der Wiedergabe des Bildes lediglich das Signal eines der beiden Kanäle verwendet.

Die Orientierung des Instruments wird über den Lagesensor in Bezug auf eine Referenz, etwa in Bezug auf einen Horizont erfasst. Bei der Referenz kann es sich um eine allgemeingültige (globale) Referenz handeln oder eine fallweise definierte Referenz. Üblicherweise wird auf einen Horizont abgestellt, der die Lage und Orientierung der beiden Augen eines Beobachters widerspiegelt.

Im ersten Wiedergabemodus werden die ersten Bilddaten und die zweiten Bilddaten derart kombiniert, dass eine Stereo-Wiedergabe (3D-Wiedergabe) ermöglicht ist. Im zweiten Wiedergabemodus werden entweder die ersten Bilddaten oder die zweiten Bilddaten verwendet, so dass eine Mono-Wiedergabe (2D-Wiedergabe) ermöglicht ist.

Zumindest gemäß beispielhaften Ausgestaltungen ist das Beobachtungsinstrument ein handgehaltenes oder handgeführtes Instrument. Dies ist jedoch nicht einschränkend zu verstehen. Gemäß einer alternativen Ausgestaltung ist das Beobachtungsinstrument an einem Stativ oder ähnlichem aufgenommen. Die Orientierung des Instruments, insbesondere dessen Rollorientierung, kann direkt über den Bediener und/oder über eine motorbetriebene Mechanik verändert werden. Das offenbarungsgemäße Verfahren kann entsprechend ausgestaltet sein.

Gemäß einem weiteren Aspekt der vorliegenden Offenbarung wird die Aufgabe der Erfindung durch ein Verfahren zur Stereo-Beobachtung nach Anspruch 14 gelöst.

Auch auf diese Weise wird die Aufgabe der Erfindung vollständig gelöst.

Das Verfahren kann auch als bildgebendes Verfahren zur Stereo-Beobachtung bezeichnet werden. Es versteht sich, dass das Verfahren gemäß den im Rahmen dieser Offenbarung beschriebenen beispielhaften Ausgestaltungen des Bildgebungssystems ausgestaltet sein kann, und umgekehrt.

Das Verfahren umfasst in einer beispielhaften Ausführungsform die Bereitstellung einer Steuereinrichtung zur Steuerung des Bildgebungssystems.

Das Verfahren umfasst in einer weiteren beispielhaften Ausführungsform des Verfahrens die Bereitstellung eines Stereo-Bildgebungssystems mit einem zur Stereo-Beobachtung ausgebildeten Beobachtungsinstrument, mit zumindest einem Lagesensor zur Erfassung einer Orientierung, insbesondere einer Drehorientierung, des Instruments, und mit einer Steuereinrichtung, die in Abhängigkeit von der Orientierung des Instruments zumindest in einem ersten Wiedergabemodus oder in einem zweiten Wiedergabemodus betreibbar ist, wobei die Steuereinrichtung zur Ausgabe eines Bildsignals ausgebildet ist, das im ersten Wiedergabemodus ein Stereo-Signal auf Basis der ersten Bilddaten und der zweiten Bilddaten umfasst, und im zweiten Wiedergabemodus ein Mono-Signal auf Basis der ersten Bilddaten oder der zweiten Bilddaten umfasst, und wobei die Steuereinrichtung dazu ausgebildet ist, mit dem Bildsignal ausgegebene Bilder im zweiten Wiedergabemodus in Abhängigkeit von der Orientierung aufzurichten.

Im ersten Wiedergabemodus werden die ausgegebenen Bilder, zumindest in beispielhaften Ausgestaltungen, nicht aufgerichtet, also unaufgerichtet ausgegeben. Demgemäß würden die angezeigten Bilder gemeinsam mit dem Rotationswinkel (Rollwinkel) des Instruments in Bezug auf die Lagereferenz rotieren.

Gemäß einer beispielhaften Ausgestaltung des Bildgebungssystems oder des Verfahrens ist die Steuereinrichtung dazu ausgebildet, ausgegebene Bilder im zweiten Wiedergabemodus auszurichten, so dass sich die Orientierung des angezeigten Bildes in Bezug auf einen als Referenzhorizont dienenden Anzeigehorizont nicht oder nur innerhalb definierter Grenzen verändert. Das Ergebnis der Ausrichtung ist dann die Wiedergabe aufgerichteter Bilder.

Auf diese Weise kann eine Bildaufrichtung realisiert werden. Im Kontext der vorliegenden Offenbarung beschreibt der Begriff Bildaufrichtung eine Funktion, bei der das wiedergegebene Bild seine Orientierung (oben - unten - rechts - links) beibehält, und zwar auch dann, wenn das Instrument gedreht wird. Insbesondere wird dabei auf eine sogenannte Rollbewegung, also auf eine Rotation der Bilderfassungseinheit bzw. einer damit gekoppelten Objektivbaugruppe abgestellt. Die Rotation definiert eine Achse für die Rollbewegung. Beispielsweise kann die Rotation bei einem Endoskop um die Längsachse eines Instrumentenschaftes erfolgen. Es sind jedoch auch flexible oder teilweise auslenkbare Beobachtungsinstrumente bekannt, bei denen keine starre Längsachse verbaut ist. Ferner sind auch Beobachtungsinstrumente mit einem Schaft und einem daran aufgenommenem Beobachtungskopf bekannt, bei denen die Rotation nicht um die Längsachse des Schaftes sondern um eine Achse durch den Beobachtungskopf erfolgt. Diese Achse ist beispielsweise senkrecht zur Längsachse des Schaftes.

Gemäß einer weiteren beispielhaften Ausgestaltung des Bildgebungssystems oder des Verfahrens ist die Steuereinrichtung dazu ausgebildet, auszugebende Bilder im ersten Wiedergabemodus unaufgerichtet auszugeben, so dass Änderungen der Orientierung des Instruments mit Änderungen der Orientierung der auszugebenden Bilder einhergehen.

Der Begriff "unaufgerichtet" wird im Rahmen der vorliegenden Offenbarung derart verstanden, dass die auszugebenden Bilder ohne Aufrichtung, also nicht aufgerichtet, ausgegeben werden. Demgemäß würde etwa ein Objekt im auszugebenden Bild bei einer Rotation des Instruments um 5° um seine Längsachse bei der Wiedergabe ebenso auch um 5° rotiert werden.

Es hat sich gezeigt, dass im ersten Wiedergabemodus unter Umständen auch leichte Drehwinkel akzeptabel sind, bei denen für den Beobachter der stereoskopische Effekt (3D-Effekt mit Tiefeneindruck) wahrnehmbar ist und gleichzeitig die Orientierung im Bild leicht fällt. Daher wird im Rahmen dieser Ausgestaltung vorgeschlagen, innerhalb begrenzter Drehwinkel (auch bezeichnet als Rollwinkel) die stereoskopische Wiedergabe im ersten Wiedergabemodus beizubehalten. Dies macht das Bildgebungssystem toleranter für leichte Drehbewegungen.

Gemäß einer weiteren beispielhaften Ausgestaltung des Bildgebungssystems oder des Verfahrens ist die Steuereinrichtung in einem ersten Drehwinkelbereich des Instruments im ersten Wiedergabemodus betreibbar, wobei die Steuereinrichtung in einem zweiten Drehwinkelbereich des Instruments im zweiten Wiedergabemodus betreibbar ist. Beispielsweise umfasst der erste Drehwinkelbereich zumindest eine Neutrallage des Instruments in Bezug auf die Lagereferenz. Ein Drehwinkelbereich ist zumindest ein definierter Unterabschnitt des zur Verfügung stehenden Drehwinkels.

Die Neutrallage umfasst beispielsweise eine parallele Ausrichtung einer Verbindungslinie zwischen den beiden Bildsensoren bzw. den Aperturen der beiden Kanäle in Bezug auf die Horizontale (oder einen künstlichen Horizont). Es versteht sich, dass die Neutrallage bzw. Ausgangslage auch fallweise definiert werden kann, über geeignete Bedienelemente.

Beispielhaft umfasst die neutrale Lage einen aufgerichteten Zustand (senkrecht zur Stereobasis) in Bezug auf die Lagereferenz (beispielsweise den Horizont). Ausgehend von der Neutrallage kann der Drehwinkel erfasst werden. Ein Rollwinkel ist üblicherweise ein Winkel, der eine Rotation um eine Längsachse (eines Schaftes und/oder der Bilderfassungseinheit) beschreibt.

Der Horizont, der die Neutrallage definiert, kann im Betrieb zumindest gemäß einer beispielhaften Ausführungsform angepasst werden. Auf diese Weise kann beispielsweise auf globale Bewegungen unter Mitnahme des Beobachtungsinstruments reagiert werden. Dies betrifft beispielsweise eine Umlagerung des Patienten unter Mitnahme des Beobachtungsinstruments. Die Anpassung des künstlichen Horizonts kann genutzt werden, um die Position und Orientierung weiterer Instrumente im wiedergegebenen Bild weitgehend beizubehalten, wenn der Patient und das Beobachtungsinstrument gedreht werden, diese weiteren Instrumente jedoch nicht.

Denkbare Drehwinkelbereiche können einerseits in Grad (in Bezug auf einen 360°-Vollkreis) oder in Stunden sowie Minuten in Bezug auf ein 12-Uhr Ziffernblatt definiert werden. Eine neutrale Lage entspricht demgemäß einer Ausrichtung von 0° bzw. 0 Uhr. Eine demgegenüber um 180° verdrehte Lage entspricht demgemäß einer Ausrichtung von 180° bzw. 6 Uhr. Zwischenpositionen für den Drehwinkel ergeben sich entsprechend. In einer beispielhaften Ausgestaltung ergänzen sich der erste Drehwinkelbereich und der zweite Drehwinkelbereich zu 360°.

Beispielhaft umfasst der erste Drehwinkelbereich einen Bereich zwischen 11 Uhr und 1 Uhr bzw. 330° und 30°. Demgemäß kann der zweite Drehwinkelbereich einen Bereich zwischen 1 Uhr und 11 Uhr bzw. zwischen 30° und 330° umfassen. Demgemäß wäre das Instrument vollständig um die Längsachse rotierbar.

Es ist jedoch auch vorstellbar, das Instrument nicht vollständig um seine Längsachse rotierbar zu gestalten (bzw. die Bildaufrichtung nur für einen solchen Teilbereich auszubilden). Beispielhaft wird ein Teilbereich zwischen 9 Uhr und 3 Uhr bzw. 270° und 90° berücksichtigt. Demgemäß kann der erste Drehwinkelbereich weiterhin beispielhaft einen Bereich zwischen 11 Uhr und 1 Uhr bzw. 330° und 30° umfassen. Jedoch weist dann der zweite Drehwinkelbereich (Teil-) Abschnitte zwischen 9 Uhr und 11 Uhr sowie zwischen 1 Uhr und 3 Uhr (entsprechend einem ersten Abschnitt zwischen 270° und 330° sowie einem zweiten Abschnitt zwischen 30° und 90°) auf.

Gemäß einer weiteren beispielhaften Ausgestaltung des Bildgebungssystems oder des Verfahrens weist der erste Drehwinkelbereich zwei zueinander um 180° versetzte Abschnitte auf. Wenn das Instrument, ausgehend von der Neutrallage, um 180° verdreht wird, ist die Bilderfassungseinheit bzw. sind die beiden Bildsensoren für die ersten und zweiten Bilddaten wiederum parallel zur Lagereferenz (Horizont) ausgerichtet. Die Bildsensoren sind seitenvertauscht. Eine solche Rollbewegung um etwa 180° wird allgemein als Flip bezeichnet. Im um 180° versetzten Abschnitt ist auch eine Stereo-Wiedergabe möglich. Dies kann auch eine (statische) Bildaufrichtung umfassen. Für eine Bildaufrichtung wäre die Bildinformation (erste und zweite Bilddaten) für den ersten Beobachtungskanal und den zweiten Beobachtungskanal zu vertauschen (rechts mit links tauschen, und umgekehrt), und die Teilbilder (rechts und links) wären jeweils für sich um 180° zu verdrehen. Gleichwohl erfolgt zusätzlich zu dieser "statischen" Bildaufrichtung keine kontinuierliche Aufrichtung bei kleinen, von 180° ausgehenden Verdrehungen.

Demgemäß umfasst der erste Drehwinkelbereich einen Bereich mit zwei Abschnitten. Ein erster Abschnitt ist beispielhaft zwischen 11 Uhr und 1 Uhr bzw. 330° und 30° gegeben. Ein zweiter Abschnitt ist beispielhaft zwischen 5 Uhr und 7 Uhr bzw. 150° und 210° gegeben. Demgemäß umfasst der zweite Drehwinkelbereich einen ersten Abschnitt und einen zweiten Abschnitt. Der erste Abschnitt umfasst einen Bereich zwischen 1 Uhr und 5 Uhr bzw. zwischen 30° und 150 °. Der zweite Abschnitt umfasst einen Bereich zwischen 7 Uhr und 11 Uhr bzw. zwischen 210 ° und 330 °. Im Bereich zwischen 11 Uhr und 1 Uhr (bzw. 330° und 30°) sowie zwischen 5 Uhr und 7 Uhr (bzw. 150° und 210°) ist sodann jeweils eine stereoskopische Beobachtung und Bildwiedergabe ermöglicht. Es versteht sich, dass die vorstehenden Angaben für den ersten Drehwinkelbereich und den zweiten Drehwinkelbereich beispielhafter Natur und nicht einschränkend zu verstehen sind. Der erste Drehwinkelbereich kann symmetrisch zur Lotrechten (0° Stellung) ausgerichtet sein. Ferner sind jedoch auch in Bezug auf die Lotrechte nichtsymmetrische Winkelbereiche vorstellbar.

Gemäß einer weiteren beispielhaften Ausgestaltung des Bildgebungssystems oder des Verfahrens ist die Steuereinrichtung dazu ausgebildet, das erste Bildsignal und das zweite Bildsignal zu tauschen und das erste Bildsignal und das zweite Bildsignal um etwa 180° zu rotieren.

Dies erfolgt vorzugsweise in dem Abschnitt des ersten Drehwinkelbereichs, der der Neutrallage um 180° versetzt gegenüberliegt. Auf diese Weise kann auch bei einem Flip eine statische Bildaufrichtung mit 3D-Funktion bereitgestellt werden.

Demgemäß umfasst der erste Wiedergabemodus gemäß diesem Ausführungsbeispiel zwei Betriebsarten. Die erste Betriebsart betrifft eine ursprüngliche Ausrichtung im Einklang mit der Lagereferenz (die beispielsweise die Neutrallage definiert). Die zweite Betriebsart betrifft den Flip-Modus (Verdrehung um 180° hierzu). Demgemäß kann der erste Abschnitt auch als Referenz-Abschnitt und der zweite Abschnitt als Flip-Abschnitt bezeichnet werden.

Gemäß einer weiteren beispielhaften Ausgestaltung des Bildgebungssystems oder des Verfahrens umfasst der zweite Drehwinkelbereich zumindest eine um 90° verdrehte Lage des Instruments gegenüber der Lagereferenz. Dies gilt beispielhaft auch für eine um 270° verdrehte Lage. Jedenfalls in einem solchen Bereich (Rollwinkel von 90° bzw. 270°) ist die Stereo-Funktionalität nicht ohne weiteres nutzbar, sofern ein aufgerichtetes Bild gewünscht ist. Daher ist es vertretbar, in diesem Bereich lediglich einen Beobachtungskanal zu nutzen, um ein 2D-Bild auszugeben. Dieses Bild kann sodann, zumindest in beispielhaften Ausführungsformen, elektronisch rotiert werden, um das Bild bedarfsweise aufzurichten.

Gemäß einer weiteren beispielhaften Ausgestaltung des Bildgebungssystems oder des Verfahrens umfasst der erste Drehwinkelbereich, bezogen auf eine Winkelskala, bei der 0° (Grad) eine ideale Ausrichtung des Instruments in Bezug auf die Lagereferenz beschreibt, einen ersten Abschnitt, der einen Bereich mit erster Grenze zwischen 310° und 350° und zweiter Grenze zwischen 10° und 50° umfasst, und wobei der erste Drehwinkelbereich vorzugsweise einen zweiten, gegenüber dem ersten Abschnitt um 180° versetzen Abschnitt aufweist.

Beispielhaft ist der erste Drehwinkelbereich symmetrisch in Bezug auf eine Vertikale (bei 0°) angeordnet. Demgemäß umfasst der erste Drehwinkelbereich beispielhaft einen Bereich zwischen +/- 10° (plus/minus 10°) und +/- 50° bezüglich der Vertikalen, deckt also bei kleinem Bereich 0° +/- 10° und bei großem Bereich 0° +/- 50° ab. Beispielhaft deckt der erste Drehwinkelbereich einen Bereich von 0° +/- 45° ab. In einem weiteren Beispiel deckt der erste Drehwinkelbereich einen Bereich von 0° +/- 30° ab. Demgemäß kann der zweite Abschnitt einen Bereich von 180° +/- 10° bis hin zu 180° +/- 50° abdecken, beispielhaft 180° +/- 30° oder 180° +/- 45°.

Absolut betrachtet erstreckt sich in einem Ausführungsbeispiel der erste Abschnitt des ersten Drehwinkelbereichs zwischen 330° und 30°, wobei sich der zweite Abschnitt des ersten Drehwinkelbereichs zwischen 150° und 210° erstreckt. Der zweite Drehwinkelbereich, in dem das Instrument im zweiten Wiedergabemodus betreibbar ist, kann sich komplementär zum ersten Drehwinkelbereich erstrecken, also beispielsweise mit einem ersten Abschnitt zwischen 30° und 150° sowie einem zweiten Abschnitt zwischen 210° und 330°.

In einem weiteren Ausführungsbeispiel erstreckt sich der erste Abschnitt des ersten Drehwinkelbereichs zwischen 315° und 45°, wobei sich der zweite Abschnitt des ersten Drehwinkelbereichs zwischen 135° und 225° erstreckt. Demgemäß umfasst der zweite Drehwinkelbereich in diesem Ausführungsbeispiel einen ersten Abschnitt zwischen 45° und 135° sowie einen zweiten Abschnitt zwischen 225° und 315°.

In einem dritten Ausführungsbeispiel erstreckt sich der erste Abschnitt des ersten Drehwinkelbereichs zwischen 345° und 15°, wobei sich der zweite Abschnitt des ersten Drehwinkelbereichs zwischen 165° und 195° erstreckt. Demgemäß umfasst der zweite Drehwinkelbereich in diesem Ausführungsbeispiel einen ersten Abschnitt zwischen 15° und 165° sowie einen zweiten Abschnitt zwischen 195° und 345°.

Es versteht sich, dass auch andere Ausführungsbeispiele für den ersten Drehwinkelbereich, also für den ersten Wiedergabemodus, und den zweiten Drehwinkelbereich, also den zweiten Wiedergabemodus, vorstellbar sind.

Der erste Drehwinkelbereich, also der Bereich mit Stereo-Wiedergabe aber ohne umfassende Bildaufrichtung, wird beispielsweise derart gewählt, dass sich der Bediener noch gut im Bild orientieren kann, was bei leichter Schrägstellung oft der Fall ist. Es ergibt sich der Vorteil der Stereo-Wiedergabe. Je nach konkretem Einsatz des Bildgebungssystems bzw. in Abhängigkeit von objektiven und subjektiven Präferenzen verschiedener Bediener können der erste Drehwinkelbereich bzw. der zweite Drehwinkelbereich definiert werden.

Es ist vorstellbar, den ersten Drehwinkelbereich fest vorzugeben. Es ist auch vorstellbar, eine geringe Anzahl an Varianten für den ersten Drehwinkelbereich (etwa +/- 30° oder +/- 45°, jeweils bezogen auf die Vertikale - bei 0° und gegebenenfalls bei 180°) vorzugeben, zwischen denen der Bediener wählen kann. Es ist auch vorstellbar, dem Bediener die Möglichkeit zu geben, den ersten Drehwinkelbereich und damit auch den zweiten Drehwinkelbereich in weiten Grenzen frei festzulegen.

Allgemein können die Abschnitte Bereiche von zumindest 355° bis 5° (also zumindest +/- 5°) sowie von zumindest 175° bis 185° umfassen. Dies umfasst Ausführungsbeispiele, bei denen der erste Winkelbereich Werte zwischen +/- 30° und +/-45° umfasst, jeweils bezogen auf eine 0° Stellung und gegebenenfalls eine 180° Stellung. Dies ist jedoch nicht einschränkend zu verstehen. Andere Winkelbereiche sind vorstellbar. Wenn einer der beiden Drehwinkelbereiche definiert ist, kann der korrespondierende andere Drehwinkelbereich hergeleitet werden. Die beiden Drehwinkelbereiche ergänzen sich in diesem Ausführungsbeispiel zu 360°. Am Vollkreis kann jeder der beiden Drehwinkelbereiche zweimal vorgesehen sein, um 180° versetzt.

Gemäß einer weiteren beispielhaften Ausführungsform umfasst der erste Drehwinkelbereich Abschnitte zwischen 315° und 45° sowie zwischen 135° und 225°. Damit umfasst der zweite Drehwinkelbereich Abschnitte zwischen 45° und 135° sowie zwischen 215° und 315°. Gemäß einer weiteren beispielhaften Ausführungsform umfasst der erste Drehwinkelbereich Abschnitte zwischen 330° und 30° sowie zwischen 150° und 210°. Damit umfasst der zweite Drehwinkelbereich Abschnitte zwischen 30° und 150° sowie zwischen 210° und 330°. Dies ist jedoch nicht einschränkend zu verstehen.

Gemäß einer weiteren beispielhaften Ausgestaltung des Bildgebungssystems oder des Verfahrens ist die Steuereinrichtung dazu betreibbar, bei einem Wechsel zwischen dem ersten Wiedergabemodus und dem zweiten Wiedergabemodus einen angepassten Übergang zu ermöglichen, wobei der Übergang vorzugsweise eine Anpassung zwischen der Orientierung des Mono-Signals im zweiten Wiedergabemodus sowie der Orientierung des Stereo-Signals im ersten Wiedergabemodus bei einem Schaltwinkel zwischen dem ersten Wiedergabemodus und dem zweiten Wiedergabemodus umfasst.

Der Übergang (auch bezeichnet als Übergangsmodus) macht den Wechsel zwischen der Stereo-Darstellung und der 2D-Darstellung harmonischer für den Bediener. Bildsignale, die im ersten Wiedergabemodus (Stereo) ausgegeben werden, sind regelmäßig für das rechte und das linke Auge separat aufbereitet. Dies kann beispielsweise Halbbilder umfassen, welche das Beobachtungsobjekt aus zwei leicht voneinander abweichenden Blickwinkeln zeigen. Beim Wechsel zwischen dem ersten Wiedergabemodus und dem zweiten Wiedergabemodus (und umgekehrt) erfolgt demgemäß ein Wechsel zwischen einer Darstellung mit zwei unterschiedlichen Halbbildern sowie einer Darstellung mit einheitlichen Bildern für das rechte und das linke Auge. Beispielsweise erfolgt beim Übergang ein bewusstes Ausblenden eines der beiden Halbbilder, woraufhin das andere Halbbild für beide Augen maßgeblich wird (und umgekehrt beim anderen Wiedergabemodus).

Gemäß einer weiteren beispielhaften Ausgestaltung des Bildgebungssystems oder des Verfahrens ist die Steuereinrichtung dazu ausgebildet, im zweiten Wiedergabemodus ausgegebene Bilder drehwinkelabhängig auszurichten, so dass vorzugsweise ein sprungarmer oder sprungfreier Übergang zwischen dem ersten Wiedergabemodus und dem zweiten Wiedergabemodus erfolgt.

Auf diese Weise wird ein stetiger oder nahezu stetiger Übergang ohne ein "springendes" Bild ermöglicht. Es gibt also vorzugsweise keine sprunghaften Änderungen der Drehorientierung des Bildes, wenn das Instrument gedreht wird und ein Wechsel zwischen dem ersten und dem zweiten Wiedergabemodus erfolgt. Mit anderen Worten ist zumindest in beispielhaften Ausführungsformen ein drehwinkelsprungarmer oder drehwinkelspungfreier Übergang zwischen dem ersten Wiedergabemodus und dem zweiten Wiedergabemodus ermöglicht. Es versteht sich, dass auch kleinere Sprünge durchaus akzeptabel und möglich sein können. Diese Sprünge sollten jedoch keine nachteiligen Auswirkungen auf die visuelle Orientierung des Beobachters im angezeigten Bild haben.

Mit dieser Gestaltung wird dem Umstand Rechnung getragen, dass zumindest in beispielhaften Ausführungsformen der erste Wiedergabemodus tolerante Winkelbereiche umfasst (etwa 330° bis 30° oder sogar größer), in denen das Bild in der Objektebene stereoskopisch erfasst und wiedergegeben wird, wobei in diesem (kleinen) Bereich auf eine strenge Aufrichtung des Bildes verzichtet wird. Stereo-Wiedergabe ist regelmäßig nur dann sinnvoll, wenn das angezeigte Bild gleichsam mit der Drehung des Instruments rotiert.

Wenn man jedoch dann den ersten Winkelbereich verlässt, in dem der erste Wiedergabemodus möglich ist, dann würde bei einem abrupten Übergang in den zweiten Wiedergabemodus gegebenenfalls eine sofortige Aufrichtung des Bildes in Bezug auf die Lagereferenz stattfinden. Dies hat jedoch zur Folge, dass das Bild aus Sicht des Beobachters "vorspringen" bzw. "zurückspringen" würde. Daher ist es zumindest in beispielhaften Ausgestaltungen angedacht, einen Übergabemodus bereitzustellen, um diesen Übergang weniger abrupt zu gestalten.

Gemäß einer weiteren beispielhaften Ausgestaltung des Bildgebungssystems oder des Verfahrens interpoliert die Steuereinrichtung im zweiten Wiedergabemodus die auszugebenden Bilder zwischen dem unaufgerichteten Zustand und dem aufgerichteten Zustand.

Auf diese Weise kann der Übergang durch geeignete Interpolationsschritte gleichmäßig und idealerweise ruckfrei oder ruckarm gestaltet werden. Dies verbessert die Wahrnehmung und vermeidet übermäßige optische Belastungen des Benutzers bei der Wahrnehmung.

In Abhängigkeit vom Drehwinkel des Instruments ändert sich mithin die Orientierung des auszugebenden Bildes, wobei gleichwohl eine Aufrichtung erfolgt. Die Interpolation der Bilder (bzw. die Interpolation der Orientierung der Bilder) trägt dazu bei, Sprünge beim Übergang zwischen dem ersten Wiedergabemodus und dem zweiten Wiedergabemodus zu vermeiden oder zumindest zu verringern. Die Interpolation erfolgt beispielsweise zwischen einer Ist-Verdrehung bei der Übergabe zwischen dem ersten Wiedergabemodus und dem zweiten Wiedergabemodus und einer Soll-Orientierung, welche dem (ideal oder nahezu ideal) aufgerichteten Bild entspricht.

Damit erfolgt unmittelbar beim Übergang zwischen dem ersten Wiedergabemodus und dem zweiten Wiedergabemodus kein "Sprung" des Bildes bzw. keine abrupte Verdrehung des Bildes. Stattdessen wird das Bild kontinuierlich bewegt bzw. verdreht. Somit nimmt der Beobachter einerseits den Wechsel zwischen dem ersten Wiedergabemodus und dem zweiten Wiedergabemodus wahr (der auch einen Wechsel zwischen 3D und 2D umfasst). Ferner kann der erste Wiedergabemodus mit der stereoskopischen Wiedergabe hinreichend tolerant gestaltet werden, so dass der Stereo-Effekt nicht nur bei absolut genauer Ausrichtung der Bilderfassungseinheit in Bezug auf die Lagereferenz möglich ist.

Gemäß einer weiteren beispielhaften Ausgestaltung des Bildgebungssystems oder des Verfahrens ist die Steuereinrichtung dazu ausgebildet, auszugebende Bilder im zweiten Wiedergabemodus zwischen einem Schaltwinkel, der dem Übergang zwischen dem ersten Wiedergabemodus und dem zweiten Wiedergabemodus zugeordnet ist, und bei dem das Bild insbesondere eine dem Schaltwinkel entsprechende Orientierung aufweist, und einem Grenzwinkel oder Grenzwinkelbereich des Instruments im zweiten Wiedergabemodus aufzurichten.

Beispielhaft beschreibt der Grenzwinkel einen Bereich, in dem das wiedergegebene Bild vollständig aufgerichtet ist, bei dem also die Orientierung des wiedergegebenen Bildes mit der Soll-Orientierung, definiert durch die Lagereferenz bzw. einen gewählten künstlichen Horizont, übereinstimmt. Beispielhaft wird der Grenzwinkel einer Drehorientierung des Instruments von 90° sowie von 270° zugeordnet. Es kann also zwei Grenzwinkel geben. Es ist auch vorstellbar, die vollständige Aufrichtung des Bildes in einem Grenzwinkelbereich anzubieten, also nicht nur bei einer bestimmten Winkelposition. Ein solcher Grenzwinkelbereich kann beispielhaft einen Bereich von 90° +/- 15° abdecken. Ein komplementärer, zweiter Grenzwinkelbereich kann beispielhaft einen Bereich von 270° +/- 15° abdecken. Die bedeutet, dass das wiedergegebene Bild bei Drehung des Instruments in diesem Bereich seine Aufrichtung nicht oder nur unwesentlich ändert.

Gemäß einer weiteren beispielhaften Ausgestaltung des Bildgebungssystems oder des Verfahrens liegt der Schaltwinkel, bezogen auf eine Winkelskala, bei der 0° eine ideale Ausrichtung des Instruments in Bezug auf die Lagereferenz beschreibt, bei 25° bis 50°, vorzugsweise bei 30° bis 45°. Bei einer symmetrischen Ausbildung des ersten Winkelbereichs gibt es demgemäß symmetrisch zur Vertikalen einen weiteren Schaltwinkel, beispielsweise im Bereich zwischen 310° und 335°, vorzugsweise bei 315° bis 330°. Die Schaltwinkel entsprechen in einer beispielhaften Ausführungsform den Grenzen des ersten Drehwinkelbereichs im ersten Wiedergabemodus.

Gemäß einer weiteren beispielhaften Ausgestaltung des Bildgebungssystems oder des Verfahrens ist die Steuereinrichtung dazu ausgebildet, auszugebende Bilder im zweiten Wiedergabemodus zwischen dem Grenzwinkel oder Grenzwinkelbereich und einem weiteren Schaltwinkel derart zu verdrehen, dass die Orientierung des angezeigten Bildes an den weiteren Schaltwinkel angepasst wird, wenn das Instrument zum weiteren Schaltwinkel hin verdreht wird.

Der weitere Schaltwinkel beschreibt den Übergang zwischen dem zweiten Wiedergabemodus und dem ersten Wiedergabemodus in einer Über-Kopf-Ausrichtung des Instruments, wobei angesichts der zumindest näherungsweise um 180° verdrehten Lage des Instruments für die stereoskopische Wiedergabe das erste Bildsignal und das zweite Bildsignal getauscht und jeweils um 180° rotiert werden, zumindest in einer beispielhaften Ausgestaltung. Das Bild steht also grundsätzlich aufrecht, wobei gewisse Drehwinkel für eine robuste Stereo-Wiedergabe toleriert werden.

In einer beispielhaften Ausführungsform wird das wiedergegebene Bild folglich nicht in einem über den (ersten) Schaltwinkel hinaus verdrehten Zustand gezeigt. In einem ersten Ausführungsbeispiel beinhaltet dies, dass das wiedergegebene Bild sowohl im ersten Wiedergabemodus als auch im zweiten Wiedergabemodus lediglich in einem innerhalb +/- 45° verdrehten Zustand gezeigt wird. In einem weiteren Ausführungsbeispiel wird das wiedergegebene Bild sowohl im ersten Wiedergabemodus als auch im zweiten Wiedergabemodus lediglich in einem innerhalb +/- 30° verdrehten Zustand gezeigt.

Gemäß einer weiteren beispielhaften Ausgestaltung des Bildgebungssystems oder des Verfahrens liegt der weitere Schaltwinkel, bezogen auf eine Winkelskala, bei der 0° eine ideale Ausrichtung des Instruments in Bezug auf die Lagereferenz beschreibt, bei 130° bis 155°, vorzugsweise bei 135° bis 150°, wobei der Grenzwinkel bei etwa 90° liegt. Bei einer symmetrischen Ausrichtung des ersten Winkelbereichs gibt es demgemäß symmetrisch zur Vertikalen einen weiteren Schaltwinkel, beispielsweise im Bereich zwischen 205° und 230°, vorzugsweise bei 210° bis 225°.

Gemäß einer weiteren beispielhaften Ausgestaltung des Instruments erlaubt die Steuereinrichtung eine Veränderung der Lagereferenz, insbesondere eine Veränderung gegenüber einer idealen Lagereferenz, die durch die Orientierung der Bilderfassungseinheit vorgegeben ist. Üblicherweise ergibt sich die Lagereferenz durch die Anordnung der beiden Aperturen der Bilderfassungseinheit. Beispielsweise befindet sich das Instrument in einer idealen Ausrichtung, in der ein Beobachter ein aufrecht stehendes Bild stereoskopisch wahrnehmen kann, wenn die beiden Aperturen in einer horizontalen Ebene zueinander versetzt angeordnet sind (horizontale Ausrichtung der Stereobasis).

Demgemäß wäre diese Ausrichtung auch die Referenz für die Bildaufrichtung im zweiten Wiedergabemodus. Es sind jedoch auch Anwendungsfälle bekannt, bei denen eine Anpassung der Lagereferenz gewünscht ist. Dies kann etwa dann der Fall sein, wenn das Beobachtungsobjekt (zum Beispiel der Patient) umgelagert wird, aber wenn gleichzeitig die Zuordnung zwischen dem Beobachtungsobjekt und dem vom Beobachtungsinstrument ausgegebenen (aufgerichteten) Bild beibehalten werden sollte. Dann kann ein künstlicher Horizont definiert werden, der sich durch einen gewählten Versatz (Versatzwinkel) gegenüber dem ursprünglichen Horizont (zum Beispiel in der idealen Ausrichtung) ergibt.

In einem solchen Betriebsmodus mit angepasstem künstlichen Horizont kann nunmehr der neu eingestellte künstliche Horizont die neue Lagereferenz für die Ausrichtung des Instruments darstellen. Im zweiten Wiedergabemodus können die wiedergegebenen Bilder unter Beachtung der neuen Lagereferenz aufgerichtet werden. Es versteht sich, dass im ersten Wiedergabemodus bei geändertem künstlichen Horizont gleichwohl die physische Ausrichtung der Bilderfassungseinheit nicht verändert wird. Bei einem Wechsel vom zweiten Wiedergabemodus hin zum ersten Wiedergabemodus erfolgt ein Übergang zwischen dem aufgerichteten 2D-Bild hin zum nicht aufgerichteten stereoskopischen Bild. Das Bild würde also "vor" oder "zurück" springen, wobei der Übergang wie vorstehend geschildert fließend gestaltet werden kann. Ob das Bild "vor" oder "zurück" springt, ist von der Drehrichtung abhängig. Ähnliches kann sich ergeben, wenn der erste Wiedergabemodus neben dem ersten Abschnitt (Neutrallage) auch einen um 180° hierzu versetzten zweiten Abschnitt (Flip-Lage) umfasst.

Zur Erfassung der Drehlage/Orientierung der Bilderfassungseinheit ist beispielhaft zumindest ein Lagesensor vorgesehen. Der Lagesensor erfasst beispielhaft eine Rollposition (Drehung um die Längsachse) des Beobachtungsinstruments und/oder der Bilderfassungseinheit. Bei einem Stereo-Endoskop erfasst der Lagesensor beispielhaft eine Rollposition des Instrumentenschaftes.

Es sind verschiedene Ausgestaltungen des Lagesensors vorstellbar. Dieser kann einen oder mehrere Beschleunigungssensoren umfassen. Beschleunigungssensoren können Relativpositionen erfassen. Zur Erfassung einer absoluten Position/Orientierung bieten sich beispielhaft Gyroskope o. ä. Sensoren an. Es versteht sich, dass der Lagesensor zumindest einen diskreten Sensor umfassen kann. Der Lagesensor ist in beispielhaften Ausführungsformen im Instrument angeordnet. Der Lagesensor ist beispielsweise im Schaft des Instruments,im Handgriff oder im Beobachtungskopf angeordnet.

Der Lagesensor kann beim proximalen Ende oder beim distalen Ende des Instruments angeordnet sein. Wenn mehrere Sensoren verbaut sind, können diese räumlich verteilt sein. Eine räumliche Verteilung erlaubt die Erfassung der Lage bzw. der Orientierung unter Berücksichtigung der Relativorientierung zwischen den Lagesensoren.

Es ist jedoch auch vorstellbar, den Lagesensor zumindest teilweise softwaretechnisch auszugestalten. Bei dem Lagesensor muss es sich also nicht unbedingt um einen diskreten Sensor handeln. Der Lagesensor kann auch ein am oder im Instrument befindlicher Tracker sein, der von einem externen Navigationssystem optisch, über Magnetfeldänderungen oder direkte elektro-magnetische Signalübertragungen erfasst wird und die Berechnung der Lage des Instruments ermöglicht. Eine Lage des aufgenommenen und wiedergegebenen Bildes kann auch über Bildverarbeitungsvorgänge erfasst werden, insbesondere wenn eine Bewegung verfolgt wird. Dies kann beispielsweise eine Mustererkennung umfassen, so dass im Bild eine Lage bestimmt und diese bei der Bewegung des Instruments bzw. der Bilderfassungseinheit stetig beibehalten wird, zumindest im zweiten Wiedergabemodus.

Gemäß einer weiteren beispielhaften Ausgestaltung des Bildgebungssystems oder des Verfahrens ist das Beobachtungsinstrument als Instrument mit geneigter Blickrichtung ausgebildet. Im Sinne der vorliegenden Offenbarung ist unter einer geneigten Blickrichtung beispielsweise eine Neigung einer optischen Achse des Objektivs in Bezug auf eine Längsachse (Drehachse) des Schaftes zu verstehen.

Es sind Instrumente mit beispielsweise um 10°, 15°, 20° 30° oder 45° geneigter Blickrichtung bekannt. Derartige Neigungswinkel sind auch im Rahmen der vorliegenden Offenbarung vorstellbar. Dies ist jedoch nicht einschränkend zu verstehen. Zumindest in beispielhaften Ausgestaltungen umfasst der Begriff geneigte Blickrichtung keine absolut seitliche Blickrichtung (90° gegenüber der Längsachse verkippt). Derartige Instrumente werden als Seitenblickinstrumente bezeichnet.

Es sind auch Instrumente mit variabler Blickrichtung bekannt, bei denen ein Neigungswinkel in Bezug auf die Längsachse verstellbar ist. Ein Instrument mit geneigter Blickrichtung kann auch als Schrägblick-Instrument bezeichnet werden. Der Neigungswinkel der Blickrichtung umfasst beispielhaft Bereiche zwischen 10° und 60° in Bezug auf die Längsachse.

Instrumente mit geneigter Blickrichtung bringen bei der Rollbewegung um die Schaftachse besondere Herausforderungen mit sich, wenn es auf die stereoskopische Beobachtung ankommt. Instrumente mit geneigter Blickrichtung werden häufig bewusst um die Schaftachse verdreht, um das aktuelle Sichtfeld zu variieren bzw. die Darstellung von Objekten im Sichtfeld zu verändern. Auf diese Weise können abhängig vom jeweiligen Rollwinkel verschiedene Bereiche bzw. Beobachtungsobjekte beobachtet werden. Dies bedeutet jedoch zumindest in beispielhaften Ausführungsformen, dass bestimmte Abschnitte der Drehbereiche stereoskopisch erfasst werden können, wogegen andere Abschnitte nur monoskopisch erfasst und wiedergegeben werden können, jedenfalls dann, wenn eine Bildaufrichtung gewünscht ist.

Wenn nämlich bei der stereoskopischen Beobachtung die Einzelbilder jeweils für sich aufgerichtet werden, dann müsste bei einer Drehung (Rollbewegung) des Instruments auch die Orientierung der Stereobasis in Abhängigkeit vom Drehwinkel angepasst werden, damit eine stereoskopische Beobachtung mit dem rechten und linken Auge möglich ist. Demgemäß wäre eine zusätzliche kombinierte, interne Schwenkbewegung der Bildsensoren bzw. der Objektive der Bilderfassungseinheit erforderlich, damit die Stereobasis an die gewünschte Bildorientierung bei der Wiedergabe und die gegebene Drehorientierung der Bilderfassungseinheit angepasst wird. Dies ist jedoch mechanisch kaum umsetzbar. Zusätzlich erschwert die geneigte Blickrichtung die Komposition eines aufgerichteten Stereo-Bildes bei beliebigen Drehorientierungen der Bilderfassungseinheit.

Gemäß einer weiteren beispielhaften Ausgestaltung des Bildgebungssystems oder des Verfahrens trägt das Beobachtungsinstrument die Bilderfassungseinheit, wobei die Bilderfassungseinheit einen Stereo-Bildsensor oder zwei zueinander versetzte Einzelsensoren aufweist.

Die Sensoren der Bilderfassungseinheit sind beispielhaft am oder bei dem distalen Ende des Instruments bzw. von dessen Schaft angeordnet. Gemäß einer alternativen Ausgestaltung sind die Sensoren der Bilderfassungseinheit am oder bei dem proximalen Ende des Instruments bzw. von dessen Schaft angeordnet.

Gemäß einer weiteren beispielhaften Ausgestaltung weist das Bildgebungssystem ferner eine Wiedergabeeinheit mit zumindest einer Anzeige auf, insbesondere einem 3D-Bildschirm und/oder einer 3D-Brille. Ein 3D-Brille kann beispielhaft in Kombination mit einem 3D-Bildschirm verwendet werden. Es sind jedoch auch 3D-Brillen bekannt, die eigene Anzeigen (Displays) aufweisen. Derartige Wiedergabeeinheiten werden beispielhaft als HMDs (Head Mounted Display - am Kopf aufgenommene Anzeigen) bezeichnet. Das offenbarungsgemäße Verfahren kann derartige Gerätschaften nutzen.

Vorzugsweise ist die Anzeige einerseits zur Wiedergabe im Stereo-Modus (3D-Wiedergabe) unter Nutzung der Bilddaten beider Beobachtungskanäle und andererseits auch zur Wiedergabe im Mono-Modus (2D-Wiedergabe) unter Nutzung der Bilddaten nur eines Beobachtungskanals geeignet.

Insgesamt kann das Instrument in verschiedenen globalen Betriebsmodi betrieben werden, zumindest in beispielhaften Ausführungsformen. In einer beispielhaften Ausführungsform gibt es vier globale Betriebsmodi.

In einem ersten globalen Modus kann das Instrument bedarfsweise zumindest im ersten Wiedergabemodus (Stereo-Wiedergabe) und im zweiten Wiedergabemodus (Mono-Wiedergabe) betrieben werden, um nach Möglichkeit eine Stereo-Wiedergabe und in Bereichen ohne Stereo-Wiedergabe bedarfsweise eine Mono-Wiedergabe mit Bildaufrichtung zu ermöglichen. Es versteht sich, dass auch Übergangsmodi im Einklang mit der vorliegenden Offenbarung vorstellbar sind, zumindest in beispielhaften Ausführungsformen.

In einem zweiten globalen Modus kann das Instrument im zweiten Wiedergabemodus (Mono-Wiedergabe) betrieben werden, wobei eine Bildaufrichtung erfolgt. Dies umfasst in einem Ausführungsbeispiel den Verzicht auf Stereo-Wiedergabe in diesem Modus.

In einem dritten globalen Modus kann das Instrument ebenso in einem Wiedergabemodus zur Mono-Wiedergabe (Wiedergabe nur eines Halbbildes) betrieben werden. Der dritte globale Modus umfasst jedoch keine Aufrichtung. Dies umfasst in einem Ausführungsbeispiel den Verzicht auf Stereo-Wiedergabe in diesem Modus.

In einem vierten globalen Modus kann das Instrument im ersten Wiedergabemodus betrieben werden (Stereo-Wiedergabe). Dies umfasst in einem Ausführungsbeispiel den Verzicht auf Bildaufrichtung. Dies umfasst in einem Ausführungsbeispiel den Verzicht auf Mono-Wiedergabe in diesem Modus.

Es versteht sich, dass bestimmte Ausführungsbeispiele die Nutzung von zwei, drei oder vier der globalen Modi umfassen.

Die Aufgabe der vorliegenden Offenbarung wird ferner durch ein Computerprogramm nach Anspruch 15 gelöst.

Es versteht sich, dass die vorstehend genannten und die nachstehend noch zu erläuternden Merkmale der Erfindung nicht nur in der jeweils angegebenen Kombination, sondern auch in anderen Kombinationen oder in Alleinstellung verwendbar sind, ohne den Rahmen der vorliegenden Erfindung zu verlassen.

Weitere Merkmale und Vorteile der Erfindung ergeben sich aus der nachfolgenden Beschreibung mehrerer bevorzugter Ausführungsbeispiele unter Bezugnahme auf die Zeichnungen. Es zeigen:
- Fig. 1:: eine gebrochene perspektivische rückwärtige Ansicht eines Beobachtungsinstruments in Form eines Endoskopes;
- Fig. 2:: eine gebrochene seitliche Teilansicht des Instruments gemäß Fig. 1;
- Fig. 3:: perspektivische vordere Ansicht eines Beobachtungsinstruments in Form eines Exoskopes;
- Fig. 4:: eine gebrochene seitliche Teilansicht des Instruments gemäß Fig. 3;
- Fig. 5:: eine vereinfachte, schematische Ansicht eines Bildgebungssystems mit einem Beobachtungsinstrument in Form eines Endoskopes;
- Fig. 6:: eine vereinfachte, schematische Ansicht eines Bildgebungssystems mit Stereo-Funktionalität;
- Fig. 7:: eine frontale Ansicht auf eine Bilderfassungseinheit zur Stereo-Beobachtung;
- Fig. 8:: eine weitere Ansicht der Bilderfassungseinheit gemäß Fig. 7 in einer gegenüber Fig. 7 rotierten Darstellung;
- Fig. 9 bis Fig. 14:: verschiedene vereinfachte schematische Darstellungen zur Veranschaulichung des Zusammenhangs zwischen einem Darstellungsmodus und einer aktuellen Drehorientierung eines Beobachtungsinstruments;
- Fig. 15:: eine vereinfachte schematische Darstellung zur Veranschaulichung der Bildkomposition bei der Stereo-Wiedergabe;
- Fig. 16:: eine weitere Darstellung der Anordnung gemäß Fig. 15 zur Veranschaulichung der Bildkomposition bei um 180° verdrehter Lage;
- Fig. 17:: eine weitere vereinfachte schematische Darstellung zur Veranschaulichung des Wechsels zwischen einem ersten Wiedergabemodus und einem zweiten Wiedergabemodus;
- Fig. 18:: ein schematisch vereinfachtes Blockdiagramm zur Veranschaulichung einer Ausführungsform eines Verfahrens zur Stereo-Beobachtung mit einem Beobachtungsinstrument; und
- Fig. 19:: ein weiteres schematisch vereinfachtes Blockdiagramm zur Veranschaulichung einer weiteren Ausführungsform eines Verfahrens zur Stereo-Beobachtung mit einem Beobachtungsinstrument.

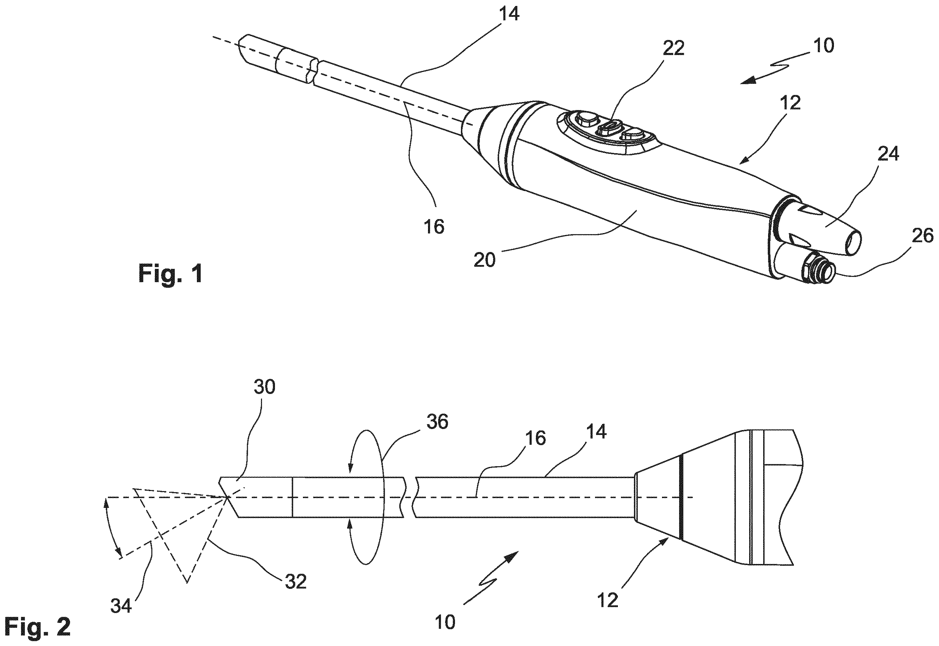

Fig. 1 veranschaulicht anhand einer perspektivischen Darstellung eine beispielhafte Ausgestaltung eines Beobachtungsinstruments 10 in Form eines Endoskopes 12. Fig. 2 zeigt eine korrespondierende seitliche Teilansicht. Das Beobachtungsinstrument 10 ist beispielhaft als Stereo-Endoskop 12 ausgestaltet. Das Endoskop 12 ist zur Beobachtung des Körperinneren ausgebildet.

Allgemein sind medizinische und nicht-medizinische (technische) Anwendungen für Endoskope 12 und ähnliche Beobachtungsinstrumente 10 bekannt. Das Endoskop 12 weist einen Schaft 14 auf, der eine Längsachse 16 definiert. Beispielhaft weist der Schaft 14 ein distales Ende und ein proximales Ende auf. Im Sinne der vorliegenden Offenbarung ist ein distales Ende ein vom Beobachter (Bediener des Instruments) abgewandtes Ende, welches dem Beobachtungsobjekt zugewandt ist. Ferner ist im Sinne der vorliegenden Offenbarung ein proximales Ende ein vom Beobachtungsobjekt abgewandtes Ende, welches dem Beobachter (Bediener des Instruments) zugewandt ist.

Am proximalen Ende des Schaftes 14 weist das Endoskop 12 ein Gehäuse 20 auf. Der Bediener kann das Endoskop 12 im Bereich des Gehäuses 20 greifen und führen. Bei der Nutzung als medizinisches Instrument 10 zur Beobachtung des Körperinneren kann der Schaft 14 des Instruments 10 zumindest teilweise in eine natürliche oder künstliche Körperöffnung eingeführt werden.

Das Gehäuse 20 weist beispielhaft Bedienelemente 22 und Anschlüsse 24, 26 auf. Die Anschlüsse 24, 26 betreffen beispielsweise eine elektrische Versorgungsleitung, eine Signalleitung, einen Beleuchtungsanschluss, sowie Anschlüsse für Flüssigkeiten oder Gase, die während der medizinischen Prozedur benötigt werden.

Fig. 2 veranschaulicht, dass das Instrument 10 beispielhaft als Schrägblick-Endoskop 12 gestaltet ist. Am distalen Ende des Schaftes 14 ist eine Bilderfassungseinheit 30 mit einem Sichtkegel/Sichtfeld 32 angedeutet. Das Sichtfeld 32 bzw. dessen Zentrum (Achse 34) ist gegenüber der Längsachse 16 geneigt. Neigungswinkel können beispielsweise 30°, 45°, 60° und dergleichen entsprechen. Auch größere oder kleinere Neigungswinkel sind denkbar. Es sind auch Schrägblick-Endoskope 12 mit verstellbarer Blickrichtung (verstellbarem Neigungswinkel) bekannt. Die Achse 34 ist beispielhaft als Normale zur Sensorfläche der Bilderfassungseinheit 30 gestaltet.

Ferner veranschaulicht in Fig. 2 ein mit 36 bezeichneter Doppelpfeil eine Rotationsbewegung des Instruments 10 um die Längsachse 16 des Schaftes 14. Eine solche Bewegung kann als Rollbewegung bezeichnet werden. Demgemäß ist die Längsachse 16 in diesem Ausführungsbeispiel eine Rollachse. Bei der Bewegung gemäß dem Pfeil 36 wird auch die gegenüber der Längsachse 16 schräg gestellte Bilderfassungseinheit 30 rotiert. Demgemäß kann durch eine solche Rollbewegung insgesamt ein deutlich größerer Bereich eines Objektfeldes observiert werden, und zwar auch dann, wenn das Instrument 10 ansonsten nicht bewegt wird.

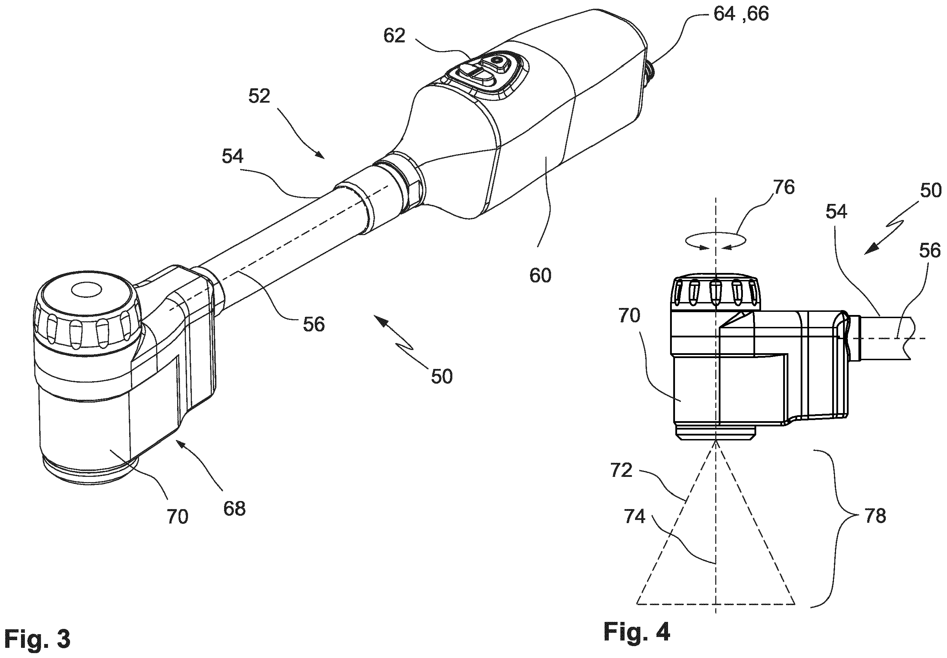

Die Figuren 3 und 4 veranschaulichen ein weiteres Beobachtungsinstrument 50, welches vorliegend als sogenanntes Exoskop 52 gestaltet ist. Beispielhaft handelt es sich bei dem Instrument 50 um ein Stereo-Exoskop 52. Das Exoskop 52 weist einen Schaft 54 mit einer Längsachse 56 auf. Am proximalen Ende des Schaftes 54 weist das Exoskop 52 ein Gehäuse 60 mit Bedienelementen 62 auf. Ferner sind am Gehäuse 60, insbesondere an dessen proximalem Ende, Anschlüsse 64, 66 ausgebildet. Auch das Exoskop 52 kann durch den Bediener im Bereich des Gehäuses 60 gehalten und auf diese Weise geführt und positioniert werden. Es ist grundsätzlich auch vorstellbar, dass Exoskop 52 an einem Stativ oder dergleichen aufzunehmen. Es kann sich dabei um ein passives Stativ (ohne die Möglichkeit zur motorischen Verstellung) oder um einen aktiven Manipulator (vergleichbar etwa einem Roboter) handeln. Jedoch ist auch die Verwendung als handgehaltenes/handgeführtes Instrument 50 denkbar.

Am distalen Ende des Schaftes 54 weist das Instrument 50 einen Beobachtungskopf 68 mit einer Bilderfassungseinheit 70 auf. Die Bilderfassungseinheit 70 weist in der beispielhaften Ausführungsform ein Sichtfeld bzw. einen Sichtkegel 72 auf, dessen Achse mit 74 bezeichnet ist. In der beispielhaften Ausführungsform gemäß den Figuren 3 und 4 ist die Achse 74 etwa senkrecht zur Längsachse 56 durch den Schaft 54 ausgerichtet. Die Achse 74 ist beispielhaft eine Normale zu einer Sensorfläche der Bilderfassungseinheit 70. Dies ist jedoch nicht einschränkend zu verstehen. Die Achse 74 kann auch eine optische Achse der optischen Anordnung der Bilderfassungseinheit 70 sein, unabhängig davon, wie der oder die Bildsensoren hierzu ausgerichtet sind.

Ferner ist in Fig. 4 mit 78 ein Arbeitsabstand angedeutet. Es versteht sich, dass der Arbeitsabstand 78 in Bezug auf die sonstigen Abmessungen des Instruments 50 nicht unbedingt maßstäblich dargestellt sein muss. Allgemein ist das Exoskop 52 gemäß den Figuren 3 und 4 im Gegensatz zu dem Endoskop 12 gemäß den Figuren 1 und 2 dazu ausgebildet, ein Objekt (Patient, technisches Objekt) von außen (außerhalb des Körpers) zu beobachten. Insofern ähneln Exoskope anderen Beobachtungsinstrumenten wie etwa Mikroskopen.

In Fig. 4 ist ferner mit 76 eine Drehbewegung/Rollbewegung der Bilderfassungseinheit 70 angedeutet. Es ist vorstellbar, das Exoskop 52 derart zu gestalten, dass die Bilderfassungseinheit 70 um die Achse 74 rotierbar ist. Bei einem Schrägblick-Instrument kann eine andere Rotationsachse gegeben sein. Bei der Ausgestaltung gemäß den Figuren 3 und 4 kann die Bilderfassungseinheit 70 im Beobachtungskopf 68 rotiert werden. Demgemäß wird nicht das Exoskop 52 in seiner Gesamtheit rotiert. Für die Rotation 76 kann einerseits eine manuelle Betätigung und andererseits eine motorische Betätigung vorgesehen sein.

Der Arbeitsabstand 78 von Exoskopen ist regelmäßig deutlich größer als derjenige von Endoskopen. Der Arbeitsabstand 78 kann beispielsweise Bereiche von 100 mm (Millimeter) bis hin zu 500 mm umfassen. Dies ist jedoch nicht einschränkend zu verstehen. Üblicherweise weisen Exoskope auch eine hinreichend große Schärfentiefe auf, die deutlich größer als die Schärfentiefe von Endoskopen ist. Beispielhaft kann die Schärfentiefe Bereiche von mindestens 5 mm, vorzugsweise von mindestens 10 mm, weiter bevorzugt von mindestens 20 mm, umfassen. Auch dies ist jedoch nicht einschränkend zu verstehen.

Beispielhaft ist ein Exoskop 52 mit einer Fokusverstellung vorgesehen. Beispielhaft ist ein Endoskop 12 mit einer festen Tiefenschärfe von 20 mm vorgesehen. Mit anderen Worten können sowohl bei Exoskopen als auch bei Endoskopen Vorrichtungen zur Anpassung der Tiefenschärfe verbaut sein. Es sind jedoch auch optische Anordnungen mit einer festen Tiefenschärfe vorstellbar.

Sowohl bei der Beobachtung mit dem Endoskop 12 als auch bei der Beobachtung mit dem Exoskop 52 ist es von Vorteil, wenn eine stereoskopische Darstellung ermöglicht ist. Eine stereoskopische Darstellung erlaubt einen Tiefeneindruck (räumlicher Eindruck) und erleichtert dabei beispielsweise die Navigation mit weiteren Instrumenten im jeweils observierten Bereich.

Demgemäß sind sowohl Endoskope 12 als auch Exoskope 52 mit 3D-Funktion (im Sinne einer stereoskopischen Beobachtung) bekannt. Üblicherweise weisen solche Instrumente dann zwei zueinander versetzte Bildsensoren oder einen Stereo-Sensor mit entsprechenden, zueinander versetzten Sensorbereichen auf. Die Stereo-Beobachtung wird durch zwei Beobachtungskanäle mit zwei voneinander beabstandeten Aperturen ermöglicht, die an das stereoskopische Sehen mit dem rechten Auge und dem linken Auge angepasst sind.

Für die Stereo-Wiedergabe sind beispielsweise sogenannte 3D-Monitore bekannt, die unter Nutzung spezieller Hilfsmittel (3D-Brille) einen stereoskopischen Effekt nutzbar machen. Ferner sind sogenannte HMDs (Head Mounted Display) bekannt, also vom Beobachter selbst getragene Videobrillen mit Stereo-Effekt.

Die stereoskopische Beobachtung und Wiedergabe stößt jedoch bei einer Rollbewegung des Instruments an ihre Grenzen, bei der sich die Stereobasis (gedachte Linie, um die die beiden Sensoren/Sensorflächen voneinander versetzt sind) gegenüber einem Referenzhorizont verändert. Dies ist insbesondere dann der Fall, wenn eine Bildaufrichtung gewünscht ist. Der Referenzhorizont wird üblicherweise durch die Anordnung einer Wiedergabeeinheit und schlussendlich durch die Anordnung der menschlichen Augen (Augenabstand, Disparität) definiert.

Wenn also die Stereobasis der Bilderfassungseinheit nicht mehr zumindest näherungsweise mit dem Referenzhorizont übereinstimmt, dann kann die stereoskopische Wiedergabe - bei Beibehaltung der Bildaufrichtung - nicht mehr mit hinreichend hoher Qualität gewährleistet werden. Dies gilt zumindest dann, wenn versucht wird, die Ausrichtung des ausgegebenen Bildes "künstlich" aufrechtzuerhalten. Die Stereo-Beobachtung ohne Bildaufrichtung ist zwar möglich, die Orientierung im nicht aufgerichteten Bild erschwert sich jedoch mit zunehmendem Drehwinkel. Es ist also bei Instrumenten zur Stereo-Beobachtung nicht einfach so möglich, Maßnahmen zur (digitalen) Bildaufrichtung zu implementieren. Das 3D-Bild wird regelmäßig auf Basis zweier Beobachtungskanäle gebildet. Eine Bildaufrichtung (digital/elektronisch oder über einen entsprechenden Aktuator zum Verdrehen des jeweiligen Sensors) könnte nun zwar das jeweilige Einzelbild im ersten Kanal und im zweiten Kanal rotieren, um dieses aufzurichten. Jedoch würde dann in großen Winkelbereichen eben keine hinreichende Übereinstimmung mehr zwischen dem Referenzhorizont und Lage der Stereobasis gegeben sein. Die vorliegende Offenbarung befasst sich mit diesem Problemkreis.

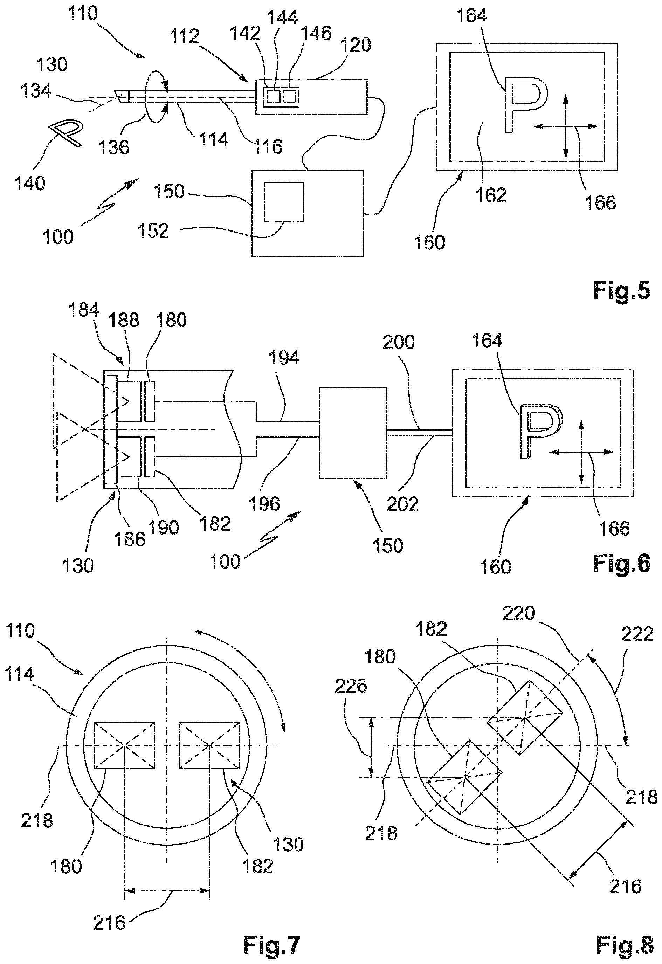

Mit Bezugnahme auf Fig. 5 und Fig. 6 wird anhand zweier schematisch stark vereinfachter Blockdarstellungen eine beispielhafte Ausgestaltung eines Bildgebungssystems 100 mit einem Beobachtungsinstrument 110 in Form eines Stereo-Endoskopes 112 veranschaulicht. Das Endoskop 112 weist einen Schaft 114 auf, der eine Längsachse 116 definiert. Am distalen Ende des Schaftes 114 sitzt eine Bilderfassungseinheit 130. In der beispielhaften Ausgestaltung gemäß Fig. 5 ist die Bilderfassungseinheit 130 derart schräg in Bezug auf den Schaft 114 orientiert, dass die Achse 134 gegenüber der Längsachse 116 geneigt ist. Das Endoskop 112 weist ferner am proximalen Ende des Schaftes 114 ein Gehäuse 120 auf. Die Bilderfassungseinheit 130 ist auf ein Beobachtungsobjekt 140 ausgerichtet. Der gekrümmte Doppelpfeil 136 in Fig. 5 veranschaulicht eine Rollbewegung des Instruments 112 um die Längsachse 116.

In der beispielhaften Ausführungsform ist im Gehäuse 120 ferner eine Sensoreinheit 142 aufgenommen, die beispielhaft einen ersten Sensor 144 aufweist. Bei dem ersten Sensor 144 handelt es sich beispielhaft um einen oder mehrere Beschleunigungssensoren. Auf diese Weise können Lageänderungen/Positionsänderungen des Instruments 110 erfasst werden. Ferner ist bei der Sensoreinheit 142 beispielhaft ein zweiter Sensor 146 vorgesehen, etwa ein Gyroskop. Die Sensoren 144, 146 können als Lagesensoren bezeichnet werden. Demgemäß kann die Sensoreinheit 142 absolut messende Lagesensoren 146 und relativ messende Lagesensoren 144 aufweisen. Es versteht sich, dass die Sensoreinheit 142 auch an anderer Position beim Instrument 112 vorgesehen sein kann, etwa nahe bei der Bilderfassungseinheit 130. Andere Ausgestaltungen der Sensoreinheit 142 mit dem zumindest einen Lagesensor 144, 146 sind ohne weiteres denkbar.

Es ist auch vorstellbar, die Sensoreinheit 142 oder zumindest einen der Lagesensoren 144, 146 außerhalb des Beobachtungsinstruments 110 zu implementieren. Beispielhaft kann eine Überwachung der Position/Orientierung des Instruments 110 auch über externe Sensoren (optisches oder elektromagnetisches Tracking von Markern, oder dergleichen) realisiert werden. Grundsätzlich ist auch eine digitale Implementation des zumindest einen Lagesensors vorstellbar. Dies kann beispielhaft Bildverarbeitungsvorgänge (Mustererkennung, Bewegungsverfolgung, etc.) umfassen. Kombinierte Gestaltungen zur Erfassung der Orientierung, insbesondere der gegenwärtigen Roll-Lage des Instruments bzw. der Bilderfassungseinheit, sind ohne weiteres denkbar.

Das Instrument 110 ist über eine Signalleitung (kabelgebunden oder kabellos) mit einer Steuereinrichtung 150 mit zumindest einer Steuereinheit 152 gekoppelt. Die Steuereinrichtung 150 kann auch zumindest teilweise in das Instrument 110 integriert sein. Die Steuereinrichtung 150 kann jedoch auch als separate Steuereinrichtung gestaltet sein. Die Steuereinrichtung 150 kann allgemein als zentrale oder dezentrale/verteilte Steuereinrichtung gestaltet sein. Die Steuereinrichtung 150 ist dazu ausgebildet, vom Instrument 110 Daten zu empfangen. Diese Daten basieren auf Bildinformationen/Bildsignalen, die mit der Bilderfassungseinheit 130 erfasst wurden. Die Steuereinrichtung 150 ist zur Datenverarbeitung ausgestaltet, insbesondere zur Verarbeitung und Aufbereitung von Bilddaten.

Eine beispielhafte Ausgestaltung einer Steuereinrichtung 150 wird von der Anmelderin unter der Bezeichnung "IMAGE1 S" als sogenannte Kameraplattform vertrieben. Üblicherweise umfasst ein Stereo-Beobachtungssystem neben dem Instrument (Endoskop oder Exoskop) eine solche Kameraplattform sowie zumindest eine Wiedergabeeinheit (etwa einen 3D-Monitor).

In der Ausgestaltung gemäß Fig. 5 ist die Steuereinrichtung 150 wiederum (Kabellos oder kabelgebunden) über eine Signalleitung mit einer Wiedergabeeinheit 160 zur Stereowiedergabe gekoppelt. Die Wiedergabeeinheit 160 ist beispielhaft als Monitor, insbesondere als 3D-Monitor gestaltet. Es versteht sich, dass unter Umständen zur Betrachtung eines 3D-Bildes (Stereo-Bildes) weitere Gerätschaften erforderlich sind, etwa eine geeignete Brille. Die Wiedergabeeinheit 160 erlaubt eine Wiedergabe des observierten Objekts 140, vergleiche das Bezugszeichen 164 zur Bezeichnung des wiedergegebenen Bildes. In Fig. 5 ist ferner mit 166 eine Lagereferenz angedeutet. Die Lagereferenz 166 umfasst zumindest einen Horizont oder ein Koordinatensystem, an dem sich das wiedergegebene Bild 162 orientiert. Bei geeigneter Ausrichtung zwischen der Bilderfassungseinheit 130, dem beobachteten Objekt 140 und dem angezeigten Bild 164 auf dem Display 162 wird eine stereoskopische Wiedergabe ermöglicht.

Es versteht sich, dass die in Fig. 5 gezeigte Anordnung ferner auch um eine Lichtquelle oder sonstige zur Verwendung mit dem Instrument 110 geeignete Einheiten ergänzt werden kann.

Fig. 6 zeigt in Ergänzung zu Fig. 5 weitere Gestaltungsmerkmale der beispielhaften Ausführungsform des Bildgebungssystems 100. Die Bilderfassungseinheit 130 ist am distalen Ende des Schaftes 114 ausgebildet. Die Bilderfassungseinheit 130 umfasst beispielhaft einen ersten Bildsensor 180 und einen zweiten Bildsensor 182. Diese sind im gezeigten Ausführungsbeispiel ebenso beim distalen Ende des Schaftes 114 angeordnet. Dies ist jedoch nicht einschränkend zu verstehen. Es sind auch Gestaltungen mit proximal angeordneten Bildsensoren denkbar.

Den Bildsensoren 180, 182 ist eine Objektivbaugruppe 184 vorgelagert. Die Objektivbaugruppe 184 umfasst beispielhaft ein Deckglas 186 und optische Einheiten 188, 190 mit Aperturen, die den Bildsensoren 180, 182 zugeordnet sind. Die optischen Einheiten 188, 190 definieren das jeweilige Sichtfeld der Bildsensoren 180, 182. Jeder der beiden Bildsensoren 180, 182 ist einem Beobachtungskanal 194, 196 zugeordnet. Einer der beiden Beobachtungskanäle 194, 196 kann als rechter Kanal und der andere als linker Kanal bezeichnet werden. Demgemäß kann einer der beiden Bildsensoren 180, 182 als rechter Sensor und der andere als linker Sensor bezeichnet werden.

Im Ausführungsbeispiel gemäß Fig. 6 bezieht sich die Bezeichnung Beobachtungskanal nicht unbedingt auf einen optischen Beobachtungskanal. Stattdessen sind die Kanäle 194, 196 regelmäßig als Kanäle zur Übermittlung von Bilddaten an die Steuereinrichtung 150 ausgebildet, die vom rechten und linken Bildsensor 180, 182 erfasst werden. Die Steuereinrichtung 150 dient zur Verarbeitung und Aufbereitung der Bilddaten. Am Ausgang der Steuereinrichtung 150 stehen ebenso zwei Ausgabekanäle 200, 202 bereit, die wiederum als rechter und linker Ausgabekanal 200, 202 bezeichnet werden können. Wenn nun über beide Ausgabekanäle 200, 202 Bildinformationen (etwa Halbbilder für das rechte und das linke Auge) bereitgestellt werden, so kann damit eine hierfür geeignete Wiedergabeeinheit 160 zur stereoskopischen Wiedergabe (3D-Wiedergabe) angesteuert werden. Fig. 6 veranschaulicht eine solche 3D-Wiedergabe des Beobachtungsobjektes 164. Das Beobachtungsobjekt 164 ist ferner in Bezug auf die Lagereferenz 166 ausgerichtet.

Fig. 7 und Fig. 8 veranschaulichen eine frontale Ansicht einer beispielhaften Ausgestaltung einer Bilderfassungseinheit 130 mit zwei Bildsensoren 180, 182. Im gezeigten Ausführungsbeispiel ist die Bilderfassungseinheit 130 im Schaft 114 eines Instruments 110 aufgenommen. Dies ist jedoch nicht einschränkend zu verstehen. Die beiden Bildsensoren 180, 182 sind um ein Maß 216 voneinander beabstandet, das an die Stereobasis (beispielsweise Abstand der Aperturen der optischen Einheiten 188, 190) angepasst ist. Die Relativlage der beiden Bildsensoren 180, 182 definiert ferner einen Sensorhorizont 218. Wenn der Sensorhorizont 218 der beiden Bildsensoren 180, 182 mit der Lagereferenz 166 (insbesondere mit dem künstlichen Horizont) übereinstimmt, so ist eine aufgerichtete 3D-Darstellung möglich.

Fig. 8 veranschaulicht einen gegenüber der Orientierung in Fig. 7 verdrehten Zustand der Bilderfassungseinheit 130. Demgemäß ist der (neue) Sensorhorizont 220 gegenüber dem alten Sensorhorizont 218 um einen bestimmten Winkel 222 verdreht. Wenn jedoch der vorherige Sensorhorizont 218 parallel zum künstlichen Horizont der Lagereferenz 166 orientiert ist, so ist dies beim neuen Sensorhorizont 220 nicht mehr der Fall. Neben der verdrehten Basis 216 wird ferner deutlich, dass ein mit 226 bezeichneter Höhenversatz gegeben ist.

Die Verdrehung/Schiefstellung in Fig. 8 erschwert die 3D-Darstellung, vor allem dann, wenn eine Bildaufrichtung gewünscht ist, also ein "aufrecht stehendes" Wiedergabebild 164 auch bei einer sich drehenden Bilderfassungseinheit 130. Dies gilt umso mehr bei Instrumenten 110 mit schräger Blickrichtung (in Bezug auf die Längsachse des Schaftes 114, die beispielhaft auch die Rollachse ist).

Die vorliegende Offenbarung adressiert diese Nachteile mit verschiedenen Wiedergabemodi bei der Darstellung. Dabei wird - sofern es möglich ist - einerseits auf die 3D-Darstellung Wert gelegt. Sofern jedoch aufgrund der gegebenen Drehlage der Bilderfassungseinheit keine 3D-Darstellung sinnvoll erscheint, wird dem Beobachter eine 2D-Darstellung angeboten. Im 2D-Modus kann wiederum die (digitale) Bildaufrichtung gewährleistet werden. Der 2D-Modus eignet sich für Anwendungsfälle, bei denen die Orientierung der Ansicht aus Sicht des Beobachters wichtiger als eine stereoskopische Beobachtung im 3D-Modus ist.

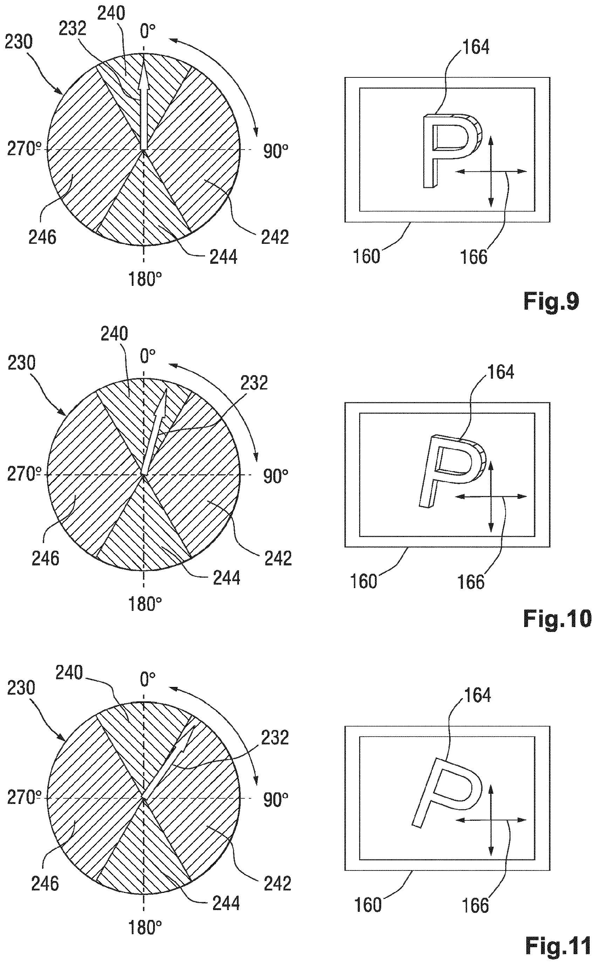

In diesem Zusammenhang wird auf die Figuren 9-14 Bezug genommen. Die Figuren 9-14 veranschaulichen anhand einer Winkelskala 230 durch einen darin eingezeichneten Positionspfeil 232 die gerade gewählte Drehorientierung des Beobachtungsinstruments 110. Ferner zeigen die Figuren 9-14 die sich hieraus ergebende Darstellung und Orientierung für das wiedergegebene Bild 164 des Beobachtungsobjekts bei der Wiedergabeeinheit 160 in Bezug auf eine Lagereferenz 166 (etwa einen künstlichen Horizont), sofern die Bildverarbeitung mit der Steuereinrichtung 150 gemäß einer beispielhaften Ausgestaltung der vorliegenden Offenbarung erfolgt. Es versteht sich, dass die Darstellungen der Kippwinkel in den Figuren 9-14 lediglich beispielhafter Natur sind. Es sind größere, aber auch kleinere Bereiche mit 3D-Darstellung vorstellbar.

Die Drehwinkelskala 230 umfasst verschiedene Bereiche 240, 242, 244, 246, von denen die Bereiche 240 und 244 beispielhaft dem ersten Wiedergabemodus mit 3D-Darstellung zugeordnet sind. Die Bereiche 242, 246 sind demgegenüber dem zweiten Wiedergabemodus mit 2D-Darstellung zugeordnet. Die Größe der jeweiligen Bereiche 240, 242, 244, 246 ist exemplarischer Natur. Zwischen den Bereichen 240, 242, 244, 246 erfolgt jeweils bei einem Schaltwinkel der Übergang zwischen den verschiedenen Wiedergabemodi.

Die 0°-Stellung entspricht beispielsweise dem in Fig. 7 gezeigten Drehwinkelzustand der Bilderfassungseinheit 130. In der 0°-Stellung entspricht der Horizont 218, der durch die beiden Sensoren 180, 182 bzw. die diesen vorgeschaltete Beobachtungsoptik definiert ist, dem künstlichen Horizont (Horizontale) der Lagereferenz 166. Damit kann ein 3D-Bild des Beobachtungsobjekts 164 ausgegeben werden. Eine Bildaufrichtung ist insoweit nicht erforderlich, als dass das Beobachtungsobjekt 164 auch ohne Zusatzmaßnahmen in der gewünschten Orientierung angezeigt wird.

Wenn nun das Instrument 110 bzw. die Bilderfassungseinheit 130 rotiert wird, so ist es bei kleinen Rotationswinkeln (vergleiche Fig. 10) noch denkbar, das Beobachtungsobjekt 164 stereoskopisch wiederzugeben (3D-Wiedergabe). Es gibt zwar keine perfekte Ausrichtung mehr zwischen dem Horizont 218 und der Lagereferenz 166. Die sich ergebende Abweichung (vergleiche beispielhaft Fig. 8) ist jedoch bei dem in Fig. 10 gezeigten Drehwinkel noch akzeptabel. Demgemäß kann das Beobachtungsobjekt 164 weiterhin stereoskopisch angezeigt werden.

In Fig. 10 wird das Beobachtungsobjekt 164 mit einem Kippwinkel im Bereich 240 angezeigt, der dem an der Skala 230 angezeigten Winkel des Zeigers 232 entspricht. Auch diese Verkippung, also der Verzicht auf die Bildaufrichtung, ist in Grenzen noch akzeptabel. Der Vorteil liegt in der Wahrung der 3D-Funktionalität. Es ist jedoch grundsätzlich auch vorstellbar, auch im ersten Wiedergabemodus (Bereiche 240, 244) das im Bild wiedergegebene Beobachtungsobjekt 164 aufzurichten, analog zur Orientierung in Fig. 9. Dies hätte einerseits den Vorteil der Bildaufrichtung. Andererseits kann dies jedoch mit weiteren Beeinträchtigungen bei der 3D-Darstellung einhergehen. Innerhalb akzeptabler Grenzen ist dies gleichwohl denkbar.

Hingegen wird in wesentlichen Ausführungsbeispielen darauf abgestellt, dass das Beobachtungsobjekt 164 zumindest im Bereich 240 nicht aufgerichtet wird. Dies ist (unbeschadet der gegebenenfalls erforderlichen Flip-Funktion) grundsätzlich auch im gegenüberliegenden Bereich 244 denkbar.

Zwischen den Darstellungen in Fig. 10 und Fig. 11 erfolgt ein Übertritt aus dem Bereich 240 in den Bereich 242. Mit anderen Worten zeigt Fig. 10 die Darstellung im ersten Wiedergabemodus (3D). Fig. 11 zeigt die Darstellung im zweiten Wiedergabemodus (2D). Wie vorstehend bereits dargelegt, ist im zweiten Wiedergabemodus die elektronische Bildaufrichtung einfacher umsetzbar.

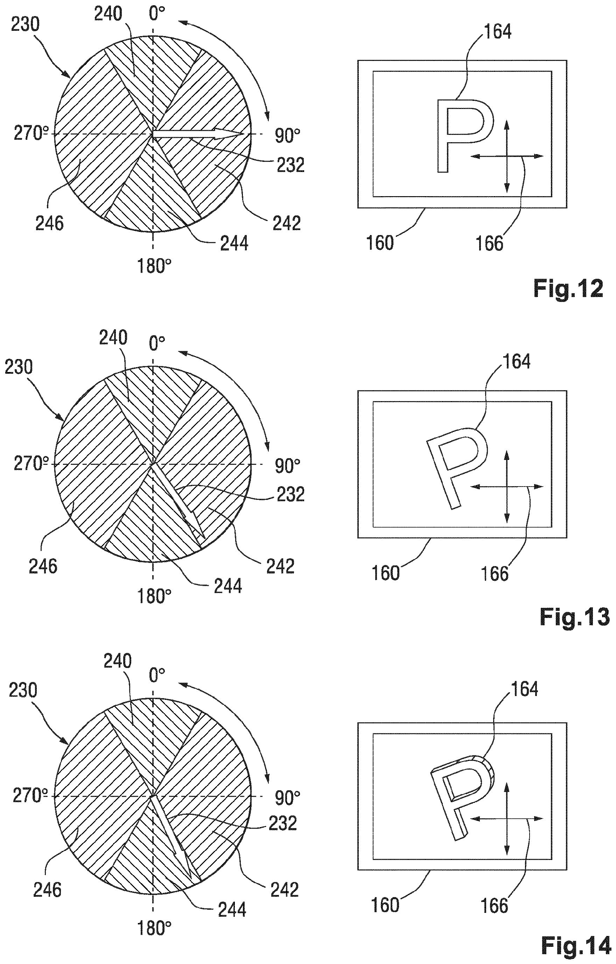

Würde man das Bild des Beobachtungsobjektes 164 etwa bei der in Fig. 11 angedeuteten Drehwinkellage abrupt beim Übergang zwischen den Bereichen 240, 242 aufrichten, so ergäbe sich ein spürbarer "Sprung", vergleichbar etwa der direkten Folge der Darstellung des Beobachtungsobjektes 164 in Fig. 10 und der Darstellung in Fig. 12. Stattdessen wird im Rahmen der vorliegenden Offenbarung, zumindest in beispielhaften Ausgestaltungen, vorgeschlagen, das Bild des Beobachtungsobjektes 164 ausgehend von der beim Wechsel vom ersten Wiedergabemodus zum zweiten Wiedergabemodus übergebenen "Orientierung" sanft und nicht abrupt aufzurichten. Eine solche Übergangsbewegung macht den Wechsel zwischen den beiden Wiedergabemodi für den Beobachter angenehmer. Mit anderen Worten findet eine Interpolation des wiedergegebenen Bildes zwischen zwei Orientierungen (teilweise verdreht sowie aufgerichtet) statt.

In ähnlicher Weise ist es beim Wechsel vom zweiten Wiedergabemodus zum ersten Wiedergabemodus vorstellbar, noch in der 2D-Darstellung die Bildaufrichtung etwas zu korrigieren, um einen sanften Übergang hin zur erwarteten Verkippung des wiedergegebenen Beobachtungsobjektes 164 beim Eintritt in den ersten Wiedergabemodus vorwegzunehmen. Hier wird das Bild zwischen der aufgerichteten Orientierung und der teilweise verdrehten Orientierung interpoliert. Vergleiche hierzu auch den weiter unten in Fig. 13 und Fig. 14 beschriebenen Übergang.

Fig. 12 veranschaulicht, dass dann innerhalb des Bereiches 242, in dem der zweite Wiedergabemodus genutzt wird, eine Bildaufrichtung ermöglicht ist. Die Bildaufrichtung kann beim 2D-Bild des Beobachtungsobjektes 164 in grundsätzlich schon bekannter Weise durchgeführt werden. So ist die digitale Bildaufrichtung bekannt. Ferner ist die Bildaufrichtung durch Bewegung des Bildsensors bekannt.

Der Zeiger 232 befindet sich in Fig. 12 bei einem Grenzwinkel im Bereich 242. Im Ausführungsbeispiel liegt der Grenzwinkel bei etwa 90°. Im gegenüberliegenden Bereich 246, der auch dem zweiten Wiedergabemodus zugeordnet ist, liegt beispielhaft ein weiterer Grenzwinkel bei etwa 270°. Zumindest dann, wenn das Instrument zum Grenzwinkel hin verdreht wurde, also etwa um 90° oder um 270°, ist im Ausführungsbeispiel eine vollständige Bildaufrichtung vorgesehen. Das Beobachtungsobjekt 164 ist beim Grenzwinkel perfekt oder zumindest näherungsweise in Bezug auf die Lagereferenz ausgerichtet. Zwischen der Orientierung beim Schaltwinkel (Übergang zwischen dem Bereich 240 und dem Bereich 242, vergleiche auch Fig. 11) und der Orientierung beim Grenzwinkel gemäß Fig. 12 kann eine allmähliche, drehwinkelabhängige Aufrichtung des Beobachtungsobjektes 164 erfolgen.

Ausgehend von der Orientierung in Fig. 12 veranschaulicht Fig. 13 einen Zustand, in dem der Zeiger 232 zwar noch im Bereich 242 ist, sich aber dem Bereich 244 nähert. Es steht also ausgehend vom zweiten Wiedergabemodus ein Übertritt in den ersten Wiedergabemodus an. Daher ist in Fig. 13 die Orientierung des wiedergegebenen Beobachtungsobjektes 164 schon an die zu erwartende Orientierung beim Eintritt in den ersten Wiedergabemodus angepasst.

Fig. 14 zeigt einen solchen Zustand. Das Instrument 110 bzw. die Bilderfassungseinheit 130 ist nahezu um 180° verdreht. Gleichwohl ist nun wieder eine 3D-Darstellung möglich. Ferner ist das wiedergegebene Beobachtungsobjekt 164 zumindest höhenrichtig in Bezug auf die ursprüngliche Anordnung (Fig. 9) ausgerichtet. Auf Basis der gegebenen Orientierung des Instruments bzw. der Bilderfassungseinheit 130 findet also in Fig. 14 ein sogenannter Image-Flip statt, umfassend einerseits eine Rotation der Einzelbilder um180° sowie andererseits einen Tausch der beiden Kanäle.

Insgesamt ergibt sich für den Beobachter auch bei einer Rollbewegung des Instruments bzw. der Bilderfassungseinheit ein einfach erfassbares Bild mit nachvollziehbarer Orientierung. Die 3D-Darstellung kann immer dann genutzt werden, wenn dies bei den gegebenen Bedingungen möglich erscheint. Es kann automatisch zwischen 3D und 2D umgeschaltet werden. Auch eine manuelle Umschaltung ist denkbar, zumindest als Zusatzoption.

Es versteht sich, dass die Folge der Darstellungen in Fig. 9 bis Fig. 14 ein Instrument mit gerader Blickrichtung betrifft. Die obigen Aussagen sind jedoch auch auf Instrumente mit schräger/geneigter Blickrichtung übertragbar. Es versteht sich, dass bei Instrumenten mit schräger Blickrichtung das Sichtfeld derart wandert, dass bei sich drehendem Instrument verschiedene Beobachtungsobjekte im Sichtfeld erscheinen. Die Ausrichtung betreffend sind jedoch die obigen Ausführungen hierauf übertragbar. Der Nutzen ergibt sich spätestens dann, wenn zusätzlich zum Beobachtungsinstrument weitere Instrumente (Zangen, Pinzetten und Ähnliches) benutzt werden und zumindest teilweise im Sichtfeld erscheinen.

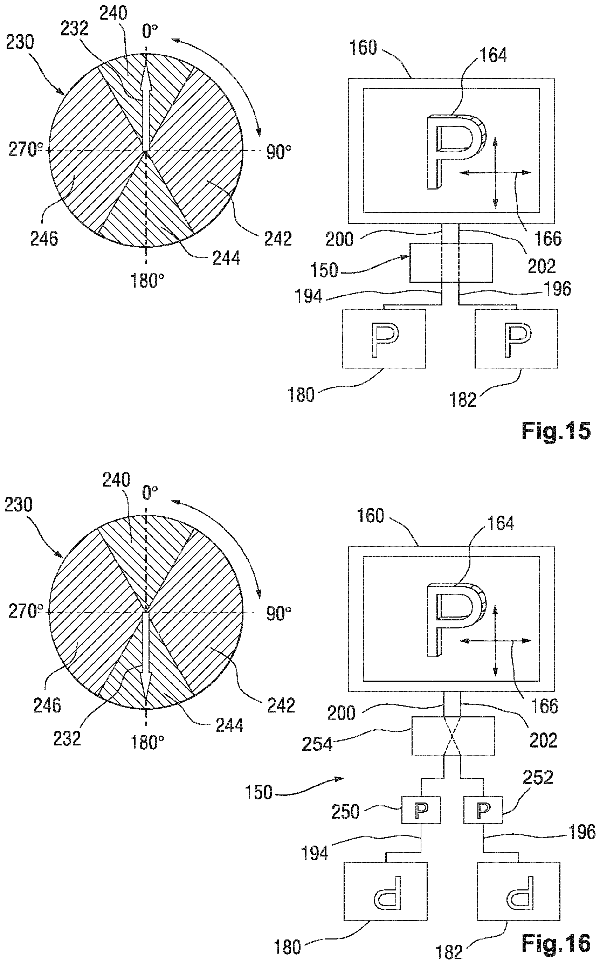

Anhand der schematisch stark vereinfachten Darstellung der Figuren 15 und 16 wird der oben schon genannte Image-Flip bei Rotation des Instruments 110 um 180° beschrieben. In Fig. 15 und Fig. 16 ist das Instrument 110 jeweils in einem der Bereiche 240, 244, in denen eine 3D-Wiedergabe sinnvoll und gewünscht ist. In Fig. 15 liegt ein Zustand vor, der etwa dem Zustand gemäß Fig. 9 entspricht. Jeder der beiden Sensoren 180, 182 erfasst ein Bild für die beiden Kanäle 194, 196. Die Steuereinrichtung 150 umfasst einen Block, der die beiden Kanäle 194, 196 schematisch den Ausgabekanälen 200, 202 zuordnet. In der Konfiguration gemäß Fig. 15 hat bereits das aufgenommene Bild der beiden Sensoren 180, 182 die gewünschte Orientierung. Demgemäß muss lediglich das Signal der beiden Sensoren 180, 182 kombiniert werden, um eine 3D-Darstellung beim wiedergegebenen Beobachtungsobjekt 164 zu ermöglichen.

Demgegenüber erfassen die beiden Sensoren 180, 182 aufgrund der zwischenzeitlichen Rotation um 180° das Bild gemäß der Konfiguration in Fig. 16 auf dem Kopf stehend. Würden nunmehr jedoch die Bilddaten beider Kanäle 194, 196 einfach nur rotiert werden (vergleiche die Blöcke 250, 252), dann wären immer noch der erste Kanal und der zweite Kanal vertauscht. Daher wird im Ausführungsbeispiel gemäß Fig. 16 vorgeschlagen, die beiden (Teil-) Bilder der beiden Sensoren 180, 182 einerseits zu rotieren, um das jeweilige Einzelbild aufzurichten. Ferner wird in diesem Ausführungsbeispiel das Signal der beiden Kanäle 194, 196 beim Verarbeiten und Weiterleiten an die Ausgabekanäle 200, 202 getauscht (über Kreuz geführt), exemplarisch veranschaulicht durch den Block 254. Auf diese Weise wird der Image-Flip (auch bezeichnet als 180°-Flip) realisiert. Im Bereich 244 ist eine näherungsweise aufgerichtete Darstellung im 3D-Modus möglich, auch wenn das Instrument 110 bzw. die Bilderfassungseinheit 130 um 180° rotiert sind. Nahe der 180°-Stellung ist auch eine 3D-Darstellung gewünscht.

Es versteht sich, dass die Blöcke 250, 252, 254 funktionale Blöcke der Steuereinrichtung 150 sein können. Die Blöcke 250, 252, 254 können softwaremäßig und/oder hardwaremäßig realisiert werden. Die Blöcke 250, 252, 254 können als diskrete Blöcke mit bestimmter Einzelfunktion oder als universale Funktionsblöcke gestaltet sein.

Die anhand der Figuren 9-16 veranschaulichte Funktonalität des Instruments 110 kann durch die Steuereinrichtung 150 in einer zugeordneten Betriebsart gesteuert werden. In einer beispielhaften Ausgestaltung ist das Instrument 110 mit der Steuereinrichtung 150 auch zu weiteren Betriebsarten befähigt.

Eine erste Betriebsart umfasst beispielsweise einen Betrieb des Instruments 110 in einem reinen 2D-Modus, also ohne stereoskopische Wiedergabe, wobei ferner auf Bildaufrichtung verzichtet wird. Das wiedergegebene Bild wird sich folglich analog zur Drehung des Instruments 110 bzw. der Bilderfassungseinheit drehen. Eine zweite Betriebsart umfasst beispielsweise einen Betrieb des Instruments 110 in einem Stereo-Modus mit stereoskopischer Wiedergabe. Damit geht einher, dass auf eine Bildaufrichtung verzichtet wird.

Eine dritte Betriebsart umfasst einen Betrieb des Instruments 110 im 2D-Modus, wobei kontinuierlich in Bezug auf einen Referenzhorizont aufgerichtet wird. Idealerweise verändert sich folglich die Drehorientierung des angezeigten Bildes nicht, wenn das Instrument gedreht wird. Der Vollständigkeit halber ist anzumerken, dass sich bei Schrägblick-Instrumenten der Bildinhalt (das Sichtfeld) ändert, wenn das Instrument rotiert wird. Jedoch behält das angezeigte Bild seine Drehorientierung. Die vierte Betriebsart ist der kombinierte Betrieb mit Stereo-Wiedergabe (erster Wiedergabemodus) und 2D-Wiedergabe (zweiter Wiedergabemodus), wobei zumindest im zweiten Wiedergabemodus das Bild zumindest teilweise aufgerichtet wird.

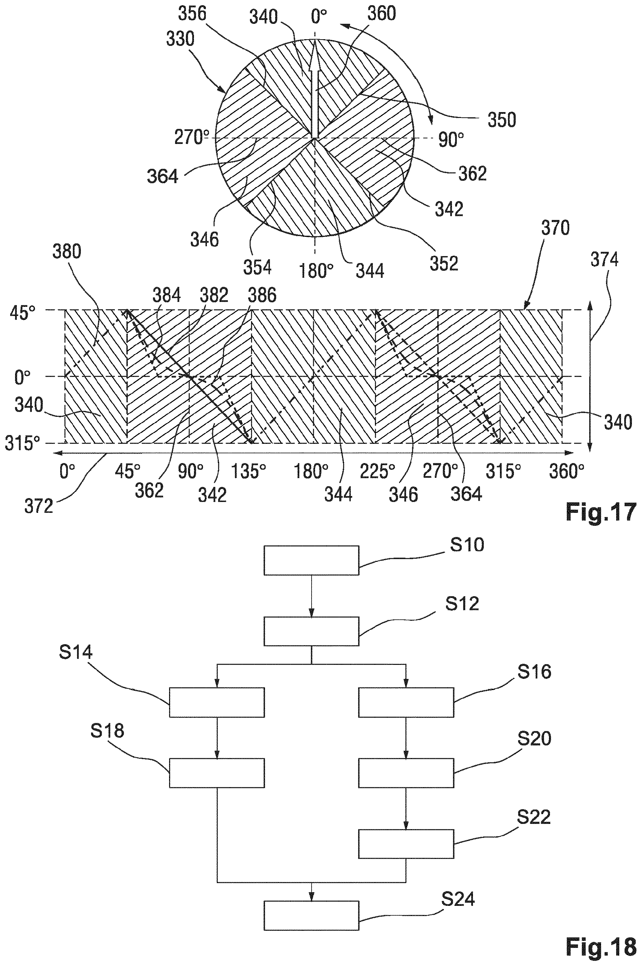

Mit Bezugnahme auf Fig. 17 wird anhand einer Winkelskala 330, welche dem Grundsatz nach der Winkelskala 230 gemäß den Figuren 9-16 entspricht, der Übergang zwischen dem ersten Wiedergabemodus und dem zweiten Wiedergabemodus sowie eine im zweiten Wiedergabemodus denkbare Bildtransformation (Interpolation) als Bestandteil der Bildaufrichtung veranschaulicht. Die Winkelskala 330 stellt einen Vollkreis dar, der einer vollständigen Rotation um die Rollachse entspricht. Es sind Teilbereiche 340, 344 vorgesehen, die dem ersten Wiedergabemodus zugeordnet sind. Ferner sind Teilbereiche 342, 346 vorgesehen, die dem zweiten Wiedergabemodus zugeordnet sind. Den jeweiligen Übergängen sind Schaltwinkel 350, 352, 354, 356 zugeordnet. Die Schaltwinkel 350, 352, 354, 356 liegen beispielsweise bei 45°, 135°, 225° sowie bei 315°. In einem alternativen, in Fig. 17 nicht dargestellten Ausführungsbeispiel liegen die Schaltwinkel 350, 352, 354, 356 bei 30°, 150°, 210° sowie bei 330°.

Der Zeiger 360 veranschaulicht die Drehlage des Instruments 110 bzw. von dessen Bilderfassungseinheit 130 bei der Rotation im ersten Wiedergabemodus (Bereiche 340, 344) und im zweiten Wiedergabemodus (Bereiche 342, 346). In Fig. 17 sind ferner Grenzwinkel 362, 364 im jeweiligen Teilbereich 342, 346 des zweiten Wiedergabemodus dargestellt. Beispielhaft liegen die Grenzwinkel 362, 364 jeweils mittig im Teilbereich 342, 346. Somit liegt der Grenzwinkel 362 beispielhaft bei 90°. Der Grenzwinkel 364 liegt beispielhaft bei 270°.

Fig. 17 enthält ferner ein korrespondierendes Diagramm 370, welches eine Abwicklung der Winkelskala 330 umfasst. Eine Achse 372 beschreibt den aktuellen Drehwinkel/Rollwinkel des Instruments 110 bzw. von dessen Bilderfassungseinheit 130 und entspricht somit der jeweiligen Position des Zeigers 360 bei der Rotation. Eine weitere Achse 374 beschreibt einen resultierenden Kippwinkel, also eine resultierende Drehorientierung des angezeigten Bildes.

In dem Ausführungsbeispiel gemäß Fig. 17 decken die Teilbereiche 340, 344 beispielhaft Bereiche von 0° +/- 45° sowie 180° +/- 45° ab. In diesen Bereichen ist eine Stereo-Wiedergabe denkbar, das Instrument ist im ersten Wiedergabemodus betreibbar. Im ersten Wiedergabemodus ist keine umfängliche elektronische Bildaufrichtung vorgesehen. Demgemäß entwickelt sich die Verkippung des angezeigten Bildes gegenüber der Lagereferenz (künstlicher Horizont) proportional oder sogar direkt proportional zum Drehwinkel des Instruments 110 bzw. von dessen Bilderfassungseinheit 130. Dies wird im Diagramm 370 durch die mit 380 bezeichnete Linie veranschaulicht, vergleiche die Abschnitte zwischen 0° und 45°, zwischen 135° und 225°, und zwischen 315° und 360°. Wenn also das Instrument 110 um 30° gegenüber der Lagereferenz verdreht ist, so ist auch das wiedergegebene Bild um 30° verdreht.

Im Abschnitt zwischen 135° und 225° ist das Instrument 110 in seiner Drehorientierung im Wesentlichen auf den Kopf gestellt, so dass das wiedergegebene Bild analog zu dem in Fig. 16 veranschaulichten Ausführungsbeispiel angepasst werden kann, um eine statische Quasi-Aufrichtung zu ermöglichen. Das Bild der beiden Beobachtungskanäle wird dann ebenso auf den Kopf gestellt, so dass das ausgegebene Bild zumindest näherungsweise die gewünschte Orientierung hat.

In den übrigen Teilbereichen 342, 346 wird hingegen eine 2D-Darstellung auf Basis eines Beobachtungskanals genutzt, vergleiche die Abschnitte zwischen 45° und 135° sowie zwischen 225° und 315°. Im zweiten Wiedergabemodus ist eine elektronische Bildaufrichtung möglich. Dies erlaubt eine Anpassung der Orientierung bzw. eine Entkopplung der Orientierung des angezeigten Bildes vom aktuellen Drehwinkel des Instruments. In beispielhaften Ausgestaltungen wird jedoch davon abgesehen, das ausgegebene Bild im zweiten Wiedergabemodus unmittelbar und konstant auf eine ideale Orientierung aufzurichten. Eine solche ideale Orientierung entspricht beispielsweise der 0°-Position auf der Achse 374 im Diagramm 370. Eine solche Funktion hätte die Folge, dass das Bild unmittelbar beim Passieren eines der Schaltwinkel 350, 352, 354, 356 sprunghaft rotiert, im Ausführungsbeispiel um 45° in Bezug auf die 0°-Auslenkung. Dies kann insbesondere bei einem Betrieb in der Nähe des jeweiligen Schaltwinkels 350, 352, 354, 356 als nachteilig empfunden werden.

Um einem solchen "Springen" des wiedergegebenen Bildes entgegenzuwirken, wird vorgeschlagen, die Orientierung des Bildes im zweiten Wiedergabemodus winkelabhängig zu interpolieren, so dass einerseits eine hinreichend stabile Aufrichtung und gute Orientierung im Bild ermöglicht ist, und andererseits Drehwinkelsprünge minimiert oder vermieden werden.

Die Kurven 382, 384, 386 veranschaulichen beispielhafte Winkelorientierungen des angezeigten Bildes in Abhängigkeit von der realen Drehorientierung des Instruments 110 bzw. der Bilderfassungseinheit 130 im zweiten Wiedergabemodus. Diese Kurven/Linien 382, 384, 386 sind in den Teilbereichen 342, 346 des zweiten Wiedergabemodus angeordnet. Die Kurven 382, 384, 386 stellen im zweiten Wiedergabemodus einen Übergang zwischen den voneinander entfernten Teilbereichen 340, 344 des ersten Wiedergabemodus bereit, insbesondere ohne Drehwinkelsprung.

Die Kurve 382 verhält sich im Wesentlichen proportional bzw. umgekehrt proportional zum aktuellen Drehwinkel des Instruments 110. Beispielsweise erstreckt sich im Diagramm 370 ein linearer Abschnitt zwischen der 45°-Position beim Schaltwinkel 350 und der 135°-Position beim Schaltwinkel 352. Mit zunehmendem Drehwinkel des Instruments 110 wird das angezeigte Bild mit zunehmendem Drehwinkel gegensinnig rotiert. Ähnlich verhält es sich im Ausführungsbeispiel zwischen den Schaltwinkeln 354 und 356, also zwischen 225° und 315°. Eine Randbedingung bei dem Ausführungsbeispiel gemäß Fig. 17 ist das Durchlaufen der 0°-Stellung bei den Grenzwinkeln 362, 364, also bei 90° und bei 270° Drehlage des Instruments. Auf diese Weise ergibt sich bei der symmetrischen, insbesondere zur Vertikalen spiegelsymmetrischen Auslegung der Winkelbereiche 340, 342, 344, 346 etwa mittig im jeweiligen Teilbereich 342, 346 des zweiten Wiedergabemodus eine vollständige Aufrichtung des angezeigten Bildes.

Die Kurve 384 ist grundsätzlich an den Verlauf der Kurve 382 angelehnt. Der Verlauf der Kurve 384 folgt jedoch dem Ziel, nicht nur direkt bei den Grenzwinkeln 362, 364 sondern auch in deren Umfeld (im Beispiel etwa +/- 20°) eine vollständige Aufrichtung des Bildes durchzuführen. Auf diese Weise ergibt sich ein Bereich, in dem das angezeigte Bild hinreichend stabil ist und nicht oder nur unwesentlich rotiert wird. Gleichwohl enthält die Kurve 384 Rampen, die einen sprungarmen Übergang zum ersten Wiedergabemodus erlauben.

Die Kurve 386 ist beispielhaft als Spline (Polynomzug) ausgebildet, wobei sich der grundsätzliche Verlauf an der Kurve 384 orientiert. Auf diese Weise können "Knicke" beim Durchlaufen der Kurve vermieden werden.

Es versteht sich, dass die Kurven 382, 384, 386 für den zweiten Wiedergabemodus mit der Kurve 380 für den ersten Wiedergabemodus kombiniert werden können, um das gewünschte Verhalten im ersten und zweiten Wiedergabemodus zu implementieren.

Mit Bezugnahme auf Fig. 18 wird anhand eines schematischen Blockdiagramms eine beispielhafte Ausführungsform eines Verfahrens zur Stereo-Beobachtung veranschaulicht, insbesondere eines Verfahrens zur Stereo-Beobachtung mit Bildaufrichtung, zumindest mit teilweiser Bildaufrichtung.

Das Verfahren umfasst einen ersten Schritt S10, der die Bereitstellung eines Beobachtungsinstruments mit Stereo-Funktionalität betrifft. Bei dem Instrument kann es sich etwa um ein Stereo-Endoskop oder um ein Stereo-Exoskop handeln. Das Instrument ist üblicherweise mit einer Bilderfassungseinheit versehen, die erste Bilddaten und zweite Bilddaten erfassen kann. Zu diesem Zweck kann die Bilderfassungseinheit einen ersten Sensor und einen hiervon versetzten zweiten Sensor aufweisen. Auf diese Weise werden zwei Beobachtungskanäle (rechts und links) gebildet. Auf diese Weise können die ersten Bilddaten und die zweiten Bilddaten zur Stereo-Beobachtung kombiniert werden. Dies bringt jedoch Herausforderungen bei der gewünschten Bildaufrichtung mit sich, wenn das Instrument rotiert wird, so dass sich die Stereobasis des Instruments gegenüber einem (idealen) Referenzhorizont verändert.

Es folgt ein Schritt S12, der eine Positionsüberwachung bzw. Erfassung einer Drehwinkellage (Roll-Lage) des Instruments bzw. von dessen Bilderfassungseinheit umfasst. Die erfasste Position bzw. die erfasste Verdrehung lässt darauf schließen, ob die gegebene Orientierung der Bilderfassungseinheit eine 3D-Darstellung ohne Bildaufrichtung sinnvoll erscheinen lässt, oder ob auf eine 2D-Darstellung unter Nutzung nur eines von beiden Beobachtungskanälen umgeschaltet werden sollte, um eine elektronische Bildaufrichtung nutzen zu können.

In Abhängigkeit von dem erfassten Drehwinkel kann ein Schritt S14 folgen, in dem ein erster Wiedergabemodus aktiviert wird. Der erste Wiedergabemodus umfasst eine 3D-Wiedergabe. Alternativ kann ein Schritt S16 folgen, in dem ein zweiter Wiedergabemodus aktiviert wird. Der zweite Wiedergabemodus umfasst eine 2D-Wiedergabe.

Dem Schritt S14 folgt ein Schritt S18, der eine Wiedergabe unter Nutzung beider Beobachtungskanäle (rechts und links) für die 3D-Wiedergabe mit Tiefeneindruck umfasst. Zumindest in beispielhaften Ausgestaltungen des Verfahrens ist vorgesehen, dass wiedergegebene Bild des Beobachtungsobjekt im ersten Wiedergabemodus nicht kontinuierlich aufzurichten. Damit verdreht sich das angezeigte Bild gemeinsam mit der Drehung des Instruments. Da jedoch der erste Wiedergabemodus nur in begrenzten Drehwinkelbereichen sinnvoll nutzbar ist, stimmt die grobe Bildausrichtung weiterhin, so dass eine Orientierung im Bild möglich ist. Gleichwohl hat der erste Wiedergabemodus den Vorteil der Stereo-Beobachtung.

Üblicherweise umfasst der erste Wiedergabemodus auch einen Zustand, in dem das Instrument bzw. dessen Bilderfassungseinheit um ca. 180° gedreht ist. Dann ist nämlich die Stereobasis (zum Beispiel definiert durch Position bzw. Orientierung der Aperturen der Beobachtungsoptik) wiederum parallel oder nahezu parallel zum Referenzhorizont ausgerichtet. Damit das angezeigte Bild jedoch nicht auf dem Kopf steht, erfolgt ein sogenannter 180°-Flip. Dies umfasst beispielhaft eine 180°-Rotation der beiden Kanäle sowie einen Tausch (rechts mit links, und umgekehrt). Auf diese Weise kann eine quasi-Aufrichtung im um 180° verdrehten Zustand gemeinsam mit der 3D-Darstellung realisiert werden. Es gibt jedoch keine kontinuierliche, eng nachgeführte Bildaufrichtung.

Wird jedoch das Verfahren gemäß dem Schritt S16 im zweiten Wiedergabemodus ausgeführt, so wird im nachfolgenden Schritt S20 nur einer der beiden Beobachtungskanäle für die Wiedergabe bereitgestellt bzw. genutzt. Dies hat den Vorteil, dass auf die Stereobasis keine Rücksicht mehr genommen werden muss, da gewissermaßen nur ein Mono-Signal für die Ausgabe aufbereitet wird.

Demgemäß kann das auszugebende Bild digital/elektronisch aufgerichtet werden. Die Bildaufrichtung erfolgt im Schritt S22. In Abhängigkeit von der weiterhin erfassten Drehwinkelposition kann nun für eine Nachführung bzw. kontinuierliche Bildaufrichtung gesorgt werden. Für den Beobachter ändert sich die allgemeine Ausrichtung des Bildes nicht oder nur innerhalb definierter Grenzen, wenn das Instrument verdreht wird.

Das Verfahren schließt im Schritt S24 mit der Wiedergabe des Bildes, im Ausführungsbeispiel entweder einer 3D-Wiedergabe ohne unmittelbare Bildaufrichtung, außer bei einer Kopfüber-Ausrichtung des Instruments, oder einer 2D-Wiedergabe mit Bildaufrichtung.

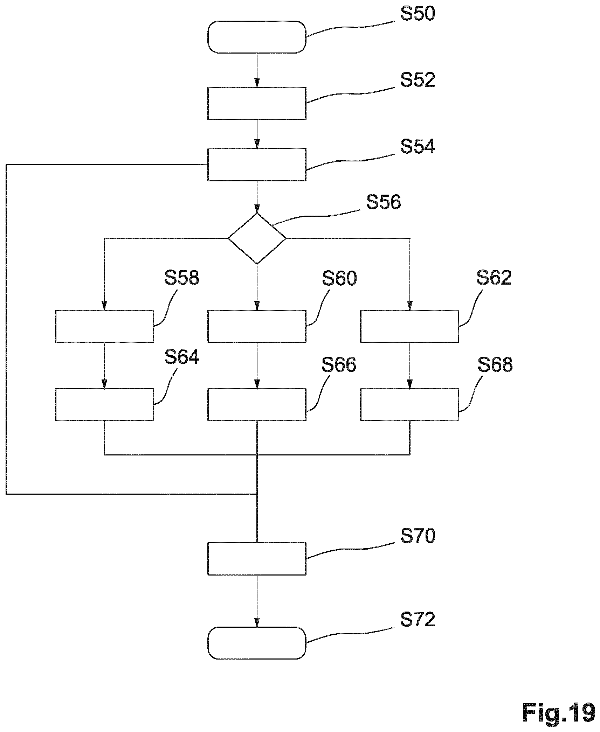

Mit Bezugnahme auf Fig. 19 wird anhand eines weiteren Blockdiagramms eine beispielhafte Ausgestaltung eines Verfahrens zur Stereo-Beobachtung beschrieben. Das Verfahren kann grundsätzlich auch als Verfahren zur Steuerung eines Bildgebungssystems gestaltet und beschrieben sein.

Das Verfahren startet mit einem Schritt S50, der beispielsweise die Aktivierung des Bildgebungssystems betrifft. Es folgt ein Schritt S52, der die Erfassung einer aktuellen Drehposition (Rollposition) eines Instruments bzw. von dessen Bilderfassungseinheit betrifft. Zu diesem Zweck kann zumindest ein entsprechender Sensor bereitgestellt werden. Die Erfassung erlaubt im Schritt S54 eine Überwachung des erfassten Winkels. Im Schritt S56 wird ermittelt, in welchem von drei (globalen) Winkelbereiche sich das Instrument bzw. dessen Bilderfassungseinheit aktuell befindet. Hierbei wird beispielsweise auf eine Lage in Bezug auf einen Referenzhorizont abgestellt, wobei die Stereobasis als Instrumenten-interne Referenz hierfür herangezogen werden kann.

In Abhängigkeit davon, welcher Winkelbereich vorliegt, kann das Instrument nachfolgend in einem ersten Wiedergabemodus (Schritt S58), einem zweiten Wiedergabemodus (Schritt S60), oder einem dritten Wiedergabemodus (Schritt S62), auch bezeichnet als Übergangsmodus, betrieben werden.

Der Schritt S58 zielt auf eine 3D-Wiedergabe unter Nutzung beider Bildkanäle ab. Der Schritt S60 zielt auf eine 2D-Wiedergabe unter Nutzung nur eines Bildkanals und vorzugsweise auch auf eine kontinuierliche Bildaufrichtung ab. Der Schritt S62 zielt darauf ab, einen Übergang zwischen dem ersten Wiedergabemodus und dem zweiten Wiedergabemodus zu ermöglichen.

Dem Schritt S58 ist ein Schritt S64 nachgelagert, der an den ersten Wiedergabemodus angepasste Bildmanipulationen zur 3D-Beobachtung umfasst. Der Schritt S64 umfasst beispielsweise einen sogenannten 180°-Flip, wodurch eine 180°-Rotation des Instruments berücksichtigt wird. Wenn nämlich das Instrument mit einer Stereobasis um ca. 180° verdreht ist, so ist grundsätzlich wieder eine 3D-Beobachtung möglich. Damit das Bild für den Beobachter aufrecht erscheint, sind Bildmanipulationen erforderlich, die im Schritt S64 durchgeführt werden. Zumindest in einer beispielhaften Ausgestaltung des Verfahrens ist es im ersten Wiedergabemodus abgesehen von dem etwaigen 180°-Flip nicht vorgesehen, das Bild digital aufzurichten.

Dem Schritt S60 ist ein Schritt S66 nachgelagert, der an den zweiten Wiedergabemodus angepasste Bildmanipulationen umfasst. Das Bild wird für die 2D-Wiedergabe bereitgestellt. Dies umfasst zumindest in einigen Ausführungsbeispielen eine kontinuierliche Bildaufrichtung, so dass das wiedergegebene Bild für den Beobachter aufrecht erscheint, auch wenn das Instrument verdreht wird. Die Bildaufrichtung kann ein statisches Ziel haben, also genau eine Ziel-Orientierung. Die Bildaufrichtung kann jedoch auch drehwinkelabhängig erfolgen, insbesondere zur Vermeidung großer Sprünge beim Wechsel zwischen dem ersten Wiedergabemodus und dem zweiten Wiedergabemodus.

Dem Schritt S62 ist ein Schritt S68 nachgelagert, der an den Übergangsmodus angepasste Bildmanipulationen umfasst. Der Übergangsmodus dient vorrangig dazu, einen sanften Übergang zwischen dem ersten Wiedergabemodus und dem zweiten Wiedergabemodus zu ermöglichen. Beispielhaft erfolgt der Übergang zwischen der Darstellung zweier leicht versetzter Halbbilder (Stereo) im ersten Wiedergabemodus und der Darstellung lediglich eines Bildes (2D), also der Daten lediglich eines Beobachtungskanals, im zweiten Wiedergabemodus. Es ist vorstellbar, eines der beiden Halbbilder im Stereo-Modus in einer bestimmten Zeitdauer (zum Beispiel definierte Anzahl an Einzelbildern) auszublenden oder einzublenden, so dass das andere der beiden Halbbilder das dominierende Bild wird. Auf diese Weise wird der Übergang zwischen 2D und 3D geglättet. Auf diese Weise werden sprunghafte Änderungen zwischen aufeinanderfolgenden Einzelbildern vermieden.

Im Schritt S68 erfolgt eine Anpassung bzw. Abwandlung der Aufrichtung, so dass das Bild für den Beobachter gleitend zwischen den verschiedenen Modi wechselt, wobei statt eines "Sprunges" eine sanfte, auf Interpolationen beruhende Transformation/Drehung sichtbar ist, welche bei der Betrachtung als angenehmer empfunden wird.

Es erfolgt beim nachgelagerten Schritt S70 eine Wiedergabe unter Berücksichtigung des jeweiligen Modus. Die Überwachung (Schritt S54) erfolgt kontinuierlich beim Betrieb des Instruments, wenn ein entsprechender Betriebsmodus gewählt ist. Der Übergangsmodus S62 erlaubt einen visuell angenehmen Übergang zwischen dem ersten Wiedergabemodus gemäß dem Schritt S58 und dem zweiten Wiedergabemodus gemäß dem Schritt S60.

Der Schritt S72 beschließt das Verfahren und umfasst beispielsweise die Deaktivierung des Bildgebungssystems.

## Patentansprüche

1. Stereo-Bildgebungssystem, insbesondere medizinisches Bildgebungssystem, das Folgendes aufweist:
- ein Beobachtungsinstrument (10, 50, 110) mit einer Bilderfassungseinheit (30, 70, 130) zur Erfassung erster Bilddaten und zweiter Bilddaten, welche zur Stereo-Beobachtung kombinierbar sind,
- zumindest einen Lagesensor (144, 146) zur Erfassung einer Orientierung, insbesondere einer Drehorientierung, des Instruments (10, 50, 110) in Bezug auf eine Lagereferenz (166), und
- eine Steuereinrichtung (150), die in Abhängigkeit von der Orientierung des Instruments (10, 50, 110) in einem ersten Wiedergabemodus und einem zweiten Wiedergabemodus betreibbar ist,
wobei die Steuereinrichtung (150) zur Ausgabe eines Bildsignals ausgebildet ist, das im ersten Wiedergabemodus ein Stereo-Signal auf Basis der ersten Bilddaten und der zweiten Bilddaten umfasst, und im zweiten Wiedergabemodus ein Mono-Signal auf Basis der ersten Bilddaten oder der zweiten Bilddaten umfasst,
wobei die Steuereinrichtung (150) dazu ausgebildet ist, mit dem Bildsignal ausgegebene Bilder im zweiten Wiedergabemodus in Bezug auf einen Referenzhorizont in Abhängigkeit von der Orientierung aufzurichten.

2. Bildgebungssystem nach Anspruch 1, wobei die Steuereinrichtung (150) dazu ausgebildet ist, ausgegebene Bilder im zweiten Wiedergabemodus auszurichten, so dass sich die Orientierung des angezeigten Bildes in Bezug auf einen als Referenzhorizont dienenden Anzeigehorizont nicht oder innerhalb definierter Grenzen verändert.

3. Bildgebungssystem nach Anspruch 1 oder 2, wobei die Steuereinrichtung (150) dazu ausgebildet ist, auszugebende Bilder im ersten Wiedergabemodus unaufgerichtet auszugeben, so dass Änderungen der Orientierung des Instruments (10, 50, 110) mit Änderungen der Orientierung der auszugebenden Bilder einhergehen.

4. Bildgebungssystem nach einem der Ansprüche 1 bis 3, wobei die Steuereinrichtung (150) in einem ersten Drehwinkelbereich des Instruments (10, 50, 110) im ersten Wiedergabemodus betreibbar ist, und wobei die Steuereinrichtung (150) in einem zweiten Drehwinkelbereich des Instruments (10, 50, 110) im zweiten Wiedergabemodus betreibbar ist.

5. Bildgebungssystem nach Anspruch 4, wobei der erste Drehwinkelbereich zwei zueinander um 180°versetzte Abschnitte aufweist, und wobei die Steuereinrichtung (150) vorzugsweise dazu ausgebildet ist, das erste Bildsignal und das zweite Bildsignal zu tauschen und das erste Bildsignal und das zweite Bildsignal um etwa 180°zu rotieren.

6. Bildgebungssystem nach einem der Ansprüche 4 oder 5, wobei der zweite Drehwinkelbereich zumindest eine um 90°verdrehte Lage des Instruments (10, 50, 110) gegenüber der Lagereferenz (166) umfasst.

7. Bildgebungssystem nach einem der Ansprüche 4 bis 6, wobei der erste Drehwinkelbereich, bezogen auf eine Winkelskala, bei der 0°eine ideale Ausrichtung des Instruments (10, 50, 110) in Bezug auf die Lagereferenz (166) beschreibt, einen ersten Abschnitt umfasst, der einen Bereich mit erster Grenze zwischen 310°und 350° und zweiter Grenze zwischen 10° und 50°umfass t, und wobei der erste Drehwinkelbereich vorzugsweise einen zweiten, gegenüber dem ersten Abschnitt um 180°versetzen Abschnitt aufweist.

8. Bildgebungssystem nach einem der Ansprüche 1 bis 7, wobei die Steuereinrichtung (150) ferner dazu betreibbar ist, bei einem Wechsel zwischen dem ersten Wiedergabemodus und dem zweiten Wiedergabemodus einen angepassten Übergang zu ermöglichen, und wobei der Übergang vorzugsweise eine Anpassung zwischen der Orientierung des Mono-Signals im zweiten Wiedergabemodus sowie der Orientierung des Stereo-Signals im ersten Wiedergabemodus bei einem Schaltwinkel zwischen dem ersten Wiedergabemodus und dem zweiten Wiedergabemodus umfasst.

9. Bildgebungssystem nach einem der Ansprüche 1 bis 8, wobei die Steuereinrichtung (150) dazu ausgebildet ist, im zweiten Wiedergabemodus ausgegebene Bilder drehwinkelabhängig auszurichten, so dass vorzugsweise ein sprungarmer oder sprungfreier Übergang zwischen dem ersten Wiedergabemodus und dem zweiten Wiedergabemodus erfolgt.

10. Bildgebungssystem nach Anspruch 9, wobei die Steuereinrichtung (150) im zweiten Wiedergabemodus die auszugebenden Bilder drehwinkelabhängig zwischen dem unaufgerichteten Zustand und dem aufgerichteten Zustand interpoliert.

11. Bildgebungssystem nach Anspruch 9 oder 10, wobei die Steuereinrichtung (150) dazu ausgebildet ist, auszugebende Bilder im zweiten Wiedergabemodus zwischen einem Schaltwinkel, der dem Übergang zwischen dem ersten Wiedergabemodus und dem zweiten Wiedergabemodus zugeordnet ist, und bei dem das Bild insbesondere eine dem Schaltwinkel entsprechende Orientierung aufweist, und einem Grenzwinkel oder Grenzwinkelbereich des Instruments im zweiten Wiedergabemodus aufzurichten, und wobei vorzugsweise der Schaltwinkel, bezogen auf eine Winkelskala, bei der 0°eine ideale Ausrichtung des Instruments (10, 50, 110) in Bezug auf die Lagereferenz (166) beschreibt, bei 25°bis 50°liegt, weiter bevorzugt bei 30° bis 45°.

12. Bildgebungssystem nach Anspruch 11, wobei die Steuereinrichtung (150) dazu ausgebildet ist, auszugebende Bilder im zweiten Wiedergabemodus zwischen dem Grenzwinkel oder Grenzwinkelbereich und einem weiteren Schaltwinkel derart zu verdrehen, dass die Orientierung des angezeigten Bildes an den weiteren Schaltwinkel angepasst ist, wenn das Instrument zum weiteren Schaltwinkel hin gedreht ist, und wobei vorzugsweise der weitere Schaltwinkel, bezogen auf eine Winkelskala, bei der 0°eine ideale Ausrichtung des Instruments (10, 50, 110) in Bezug auf die Lagereferenz (166) beschreibt, bei 130°bis 155 °liegt, weiter bevorzugt bei 135° bis 150°, wobei der Grenzwinkel bei 90° liegt.

13. Bildgebungssystem nach einem der Ansprüche 1 bis 12, wobei das Beobachtungsinstrument (10, 50, 110) die Bilderfassungseinheit (30, 70, 130) trägt, und wobei die Bilderfassungseinheit (30, 70, 130) einen Stereo-Bildsensor oder zwei zueinander versetzte Einzelsensoren (180, 182) aufweist, und/oder wobei das Beobachtungsinstrument (10, 50, 110) als Instrument mit geneigter Blickrichtung ausgebildet ist.

14. Verfahren zum Betrieb eines Stereo-Bildgebungssystems (100) nach einem der Ansprüche 1 bis 13, umfassend die folgenden Schritte:
- mittels des Lagesensors Erfassung einer Orientierung, insbesondere einer Drehorientierung, des Instruments (10, 50, 110) in Bezug auf eine Lagereferenz (166), und
- mittels der Steuereinrichtung (150) Betreiben des Bildgebungssystems (100) in Abhängigkeit von der Orientierung des Instruments (10, 50, 110) in einem ersten Wiedergabemodus oder einem zweiten Wiedergabemodus, umfassend:
im ersten Wiedergabemodus Ausgabe eines Bildsignals, das ein Stereo-Signal auf Basis der ersten Bilddaten und der zweiten Bilddaten umfasst,
im zweiten Wiedergabemodus Ausgabe eines Bildsignals, das ein Mono-Signal umfasst,
- mittels der Steuereinrichtung (150) Aufrichten der auszugebenen Bilder in Bezug auf einen Referenzhorizont ir Abhängigkeit von der erfassten Orientierung, zumindest im zweiten Wiedergabemodus.

15. Computerprogramm, das einen Programmcode aufweist, der dazu ausgebildet ist, eir Bildgebungssystem nach einem der Ansprüche 1 bis 13 dazu zu veranlassen, die Schritte des Verfahrens nach Anspruch 14 auszuführen, wenn das Computerprogramm auf der Steuereinrichtung (150) des Bildgebungssystems (100) ausgeführt wird.

## Claims

1. A stereo imaging system, in particular a medical imaging system, comprising:
- an observation instrument (10, 50, 110) comprising an image acquisition unit (30, 70, 130) for detecting first image data and second image data, which can be combined for stereo observation,
- at least one position sensor (144, 146) for detecting an orientation, in particular a rotation orientation, of the instrument (10, 50, 110) in relation to a position reference (166), and
- a control device (150) that is operable in a first representation mode and a second representation mode, depending on the orientation of the instrument (10, 50, 110),
wherein the control device (150) is configured for outputting an image signal, which, in the first representation mode, comprises a stereo signal based on the first image data and the second image data, and, in the second representation mode, comprises a mono signal based on the first image data or the second image data,
wherein the control device (150) is adapted to erect images that are output with the image signal in the second representation mode in relation to a reference horizon, depending on the orientation.

2. The imaging system according to claim 1, wherein the control device (150) is adapted to orient output images in the second representation mode so that the orientation of the displayed image in relation to a display horizon that serves as the reference horizon does not change or changes within defined limits.

3. The imaging system according to claim 1 or 2, wherein the control device (150) is adapted in the first representation mode to output images to be output in a non-erected state, so that changes in the orientation of the instrument (10, 50, 110) are associated with changes in the orientation of the images to be output.

4. The imaging system according to one of claims 1 to 3, wherein the control device (150) is operable in a first rotation angle range of the instrument (10, 50, 110) in the first representation mode, and wherein the control device (150) is operable in a second rotation angle range of the instrument (10, 50, 110) in the second representation mode.

5. The imaging system according to claim 4, wherein the first rotation angle range comprises two sections that are offset from each other by 180°, and wherein the control device (150) is preferably adapted to swap the first image signal and the second image signal and to rotate the first image signal and the second image signal by approximately 180°.

6. The imaging system according to one of claims 4 or 5, wherein the second rotation angle range comprises at least one position of the instrument (10, 50, 110) that is rotated by 90° with respect to the position reference (166).

7. The imaging system according to one of claims 4 to 6, wherein the first rotation angle range comprises, in terms of an angular scale, in which 0° indicates an ideal orientation of the instrument (10, 50, 110) in relation to the position reference (166), a first section covering a range having a first limit between 310° and 350° and a second limit between 10° and 50°, and wherein the first rotation angle range preferably comprises a second section that is offset by 180° relative to the first section.

8. The imaging system according to one of claims 1 to 7, wherein the control device (150) is further operable to enable an adapted transition when switching between the first representation mode and the second representation mode, and wherein the transition preferably comprises an adaptation between the orientation of the mono signal in the second representation mode and the orientation of the stereo signal in the first representation mode at a switching angle between the first representation mode and the second representation mode.

9. The imaging system according to one of claims 1 to 8, wherein the control device (150) is configured, in the second representation mode, to orient output images dependent on the rotation angle, so that preferably a low-skip or skip-free transition between the first representation mode and the second representation mode is provided.

10. The imaging system according to claim 9, wherein the control device (150) interpolates in the second representation mode the images to be output dependent on the rotation angle between the non-erected state and the erected state.

11. The imaging system according to claim 9 or 10, wherein the control device (150) is configured to erect images to be output in the second representation mode between a switching angle, which is associated with the transition between the first representation mode and the second representation mode, and in which in particular the image assumes an orientation corresponding to the switching angle, and a limit angle or limit angle range of the instrument in the second representation mode, and wherein preferably the switching angle, in terms of an angular scale, in which 0° indicates an ideal orientation of the instrument (10, 50, 110) in relation to the position reference (166), is between 25° and 50°, further preferred between 30° and 45°.

12. The imaging system according to claim 11, wherein the control device (150) is adapted to rotate images to be output in the second representation mode between the limit angle or limit angle range and a further switching angle such that the orientation of the displayed image is adapted to the further switching angle when the instrument is rotated towards the further switching angle, and wherein preferably the further switching angle, in terms of an angular scale, in which 0° indicates an ideal orientation of the instrument (10, 50, 110) in relation to the position reference (166), is between 130° and 155°, further preferred between 135° and 150°, wherein the limit angle is 90°.

13. The imaging system according to one of claims 1 to 12, wherein the observation instrument (10, 50, 110) carries the image acquisition unit (30, 70, 130), and wherein the image acquisition unit (30, 70, 130) comprises a stereo image sensor or two individual sensors (180, 182) offset relative to one another, and/or wherein the observation instrument (10, 50, 110) is arranged as an instrument having an inclined direction of view.

14. A method for operating a stereo imaging system (100) according to any one of claims 1 to 13, the method comprising the following steps:
- by means of the position sensor, detecting an orientation, in particular a rotation orientation, of the instrument (10, 50, 110) in relation to a position reference (166), and
- by means of the control device (150), operating the imaging system (100) depending on the orientation of the instrument (10, 50, 110) in a first representation mode or a second representation mode, comprising:
in the first representation mode, outputting an image signal comprising a stereo signal based on the first image data and the second image data,
in the second representation mode, outputting an image signal comprising a mono signal,
- by means of the control device (150), at least in the second representation mode, erecting the output images in relation to a reference horizon, depending on the detected orientation.

15. A computer program comprising a program code, which is adapted to cause an imaging system according to any one of claims 1 to 13 to perform the steps of the method according to claim 14, when the computer program is executed on the control device (150) of the imaging system (100).

## Revendications

1. Système d'imagerie stéréoscopique, notamment système d'imagerie médicale, qui possède les éléments suivants :
- un instrument d'observation (10, 50, 110) comprenant une unité d'acquisition d'images (30, 70, 130) destinée à acquérir des premières données d'image et des deuxièmes données d'image, lesquelles peuvent être combinées en vue d'une observation stéréoscopique,
- au moins un premier capteur de position (144, 146) destiné à détecter une orientation, notamment une orientation en rotation de l'instrument (10, 50, 110) par rapport à une référence de position (166) et
- un dispositif de commande (150) qui, en fonction de l'orientation de l'instrument (10, 50, 110), peut être utilisé dans un premier mode de reproduction et un deuxième mode de reproduction,
le dispositif de commande (150) étant configuré pour délivrer un signal d'image qui, dans le premier mode de reproduction, comporte un signal stéréo sur la base des premières données d'image et des deuxièmes données d'image et, dans le deuxième mode de reproduction, comporte un signal mono sur la base des premières données d'image ou des deuxièmes données d'image,
le dispositif de commande (150) étant configuré pour redresser les images délivrées avec le signal d'image dans le deuxième mode de reproduction par rapport à un horizon de référence en fonction de l'orientation.

2. Système d'imagerie selon la revendication 1, le dispositif de commande (150) étant configuré pour aligner les images délivrées dans le deuxième mode de reproduction de telle sorte que l'orientation de l'image affichée par rapport à un horizon d'affichage servant d'horizon de référence ne varie pas, ou varie au sein de limites définies.

3. Système d'imagerie selon la revendication 1 ou 2, le dispositif de commande (150) étant configuré pour délivrer les images à délivrer dans le premier mode de reproduction non alignées, de sorte que des modifications de l'orientation de l'instrument (10, 50, 110) s'accompagnent des modifications de l'orientation des images à délivrer.

4. Système d'imagerie selon l'une des revendications 1 à 3, le dispositif de commande (150) pouvant être utilisé dans une première plage angulaire de rotation de l'instrument (10, 50, 110) dans le premier mode de reproduction et le dispositif de commande (150) pouvant être utilisé dans une deuxième plage angulaire de rotation de l'instrument (10, 50, 110) dans le deuxième mode de reproduction.

5. Système d'imagerie selon la revendication 4, la première plage angulaire de rotation possédant deux portions décalées de 180° l'une de l'autre et le dispositif de commande (150) étant de préférence configuré pour échanger le premier signal d'image et le deuxième signal d'image et d'appliquer une rotation d'environ 180° au premier signal d'image et au deuxième signal d'image.

6. Système d'imagerie selon l'une des revendications 4 ou 5, la deuxième plage angulaire de rotation comprenant une position de l'instrument (10, 50, 110) tournée de 90° par rapport à la référence de position (166).

7. Système d'imagerie selon l'une des revendications 4 à 6, la première plage angulaire de rotation, par rapport à une graduation d'angle avec laquelle le 0° représente un alignement idéal de l'instrument (10, 50, 110) par rapport à la référence de position (166), comprenant une première portion, qui comporte une zone ayant une première limite entre 310° et 350° et une deuxième limite entre 10° et 50°, et la première plage angulaire de rotation possédant de préférence une deuxième portion, décalée de 180° par rapport à la première portion.

8. Système d'imagerie selon l'une des revendications 1 à 7, le dispositif de commande (150) pouvant en outre être utilisé, lors d'un changement entre le premier mode de reproduction et le deuxième mode de reproduction, pour rendre possible une transition adaptée et la transition comprenant de préférence une adaptation entre l'orientation du signal mono dans le deuxième mode de reproduction ainsi que de l'orientation du signal stéréo dans le premier mode de reproduction lors d'un angle de commutation entre le premier mode de reproduction et le deuxième mode de reproduction.

9. Système d'imagerie selon l'une des revendications 1 à 8, le dispositif de commande (150) étant configuré pour aligner les images délivrées dans le deuxième mode de reproduction en fonction de l'angle de rotation, de sorte qu'une transition de préférence à faible saut ou exempte de saut ait lieu entre le premier mode de reproduction et le deuxième mode de reproduction.

10. Système d'imagerie selon la revendication 9, le dispositif de commande (150), dans le deuxième mode de reproduction, interpolant les images à délivrer entre l'état non aligné et l'état aligné en fonction de l'angle de rotation.

11. Système d'imagerie selon la revendication 9 ou 10, le dispositif de commande (150) étant configuré pour redresser les images à délivrer dans le deuxième mode de reproduction entre un angle de commutation, qui est associé à la transition entre le premier mode de reproduction et le deuxième mode de reproduction et avec lequel l'image présente notamment une orientation correspondant à l'angle de commutation, et un angle limite une plage angulaire limite de l'instrument dans le deuxième mode de reproduction, et l'angle de commutation, par rapport à une graduation d'angle avec laquelle le 0° représente un alignement idéal de l'instrument (10, 50, 110) par rapport à la référence de position (166), étant de préférence situé dans la plage de 25° à 50°, encore de préférence de 30° à 45°.

12. Système d'imagerie selon la revendication 11, le dispositif de commande (150) étant configuré pour tourner les images à délivrer dans le deuxième mode de reproduction entre l'angle limite ou la plage angulaire limite et un angle de commutation supplémentaire de telle sorte que l'orientation de l'image affichée est adaptée à l'angle de commutation supplémentaire lorsque l'instrument est tourné en direction de l'angle de commutation supplémentaire, et l'angle de commutation supplémentaire, par rapport à une graduation d'angle avec laquelle le 0° représente un alignement idéal de l'instrument (10, 50, 110) par rapport à la référence de position (166), étant de préférence situé dans la plage de 130° à 15°, encore de préférence de 135° à 150°, l'angle limite étant de l'ordre de 90°.

13. Système d'imagerie selon l'une des revendications 1 à 12, l'instrument d'observation (10, 50, 110) portant l'unité d'acquisition d'images (30, 70, 130) et l'unité d'acquisition d'images (30, 70, 130) possédant un capteur d'image stéréoscopique ou deux capteurs individuels (180, 182) décalés l'un par rapport à l'autre, et/ou l'instrument d'observation (10, 50, 110) étant réalisé sous la forme d'un instrument avec une direction de vision inclinée.

14. Procédé pour faire fonctionner un système d'imagerie stéréoscopique (100) selon l'une des revendications 1 à 13, comprenant les étapes suivantes :
- au moyen du capteur de position, détection d'une orientation, notamment d'une orientation en rotation de l'instrument (10, 50, 110) par rapport à une référence de position (166) et
- au moyen du dispositif de commande (150), utilisation du système d'imagerie (100) en fonction de l'orientation de l'instrument (10, 50, 110) dans un premier mode de reproduction ou un deuxième mode de reproduction, comprenant :
dans le premier mode de reproduction, délivrance d'un signal d'image qui comporte un signal stéréo sur la base des premières données d'image et des deuxièmes données d'image,
dans le deuxième mode de reproduction, délivrance d'un signal d'image qui comporte un signal mono
- au moyen du dispositif de commande (150), redressement des images à délivrer par rapport à un horizon de référence en fonction de l'orientation, au moins dans le deuxième mode de reproduction.

15. Programme informatique, qui possède un code de programme qui est configuré pour amener un système d'imagerie selon l'une des revendications 1 à 13 à exécuter les étapes du procédé selon la revendication 14 lorsque le programme informatique est exécuté sur le dispositif de commande (150) du système d'imagerie (100).
